# EUROPEAN PATENT APPLICATION

(11) **EP 4 804 196 A2**
(43) Date of publication of application: **09.09.2026**
(21) Application number: 26194982.0
(22) Date of filing: 26.06.2023
(51) Int. Cl.: G16B 30/20

(54) **IMPROVED HUMAN LEUKOCYTE ANTIGEN (HLA) GENOTYPING**

(30) Priority: 27.06.2022 US 202263367107 P
(62) Document number(s) of the earlier application(s) in accordance with Art. 76 EPC: 23744990.5 / 4 544 554
(71) Applicant: Illumina, Inc., San Diego, CA 92122 (US)
(72) Inventor: FILIP, Ioan, San Diego, 92122 (US); ZHAO, Chen, San Diego, 92122 (US); BIRNBAUM, Adam, San Diego, 92122 (US); CHEN, Brian, San Diego, 92122 (US); RUEHLE, Michael, San Diego, 92122 (US); TRUONG, Sean, San Diego, 92122 (US)
(74) Representative: Robinson, David Edward Ashdown

(57) **Abstract**

This disclosure describes methods, non-transitory-computer readable media, and systems that can accurately genotype one or more human leukocyte antigen (HLA) alleles from a genomic sample by using alignment-score-based filtering and read-support-equivalence grouping of reads for genotype inference. To genotype HLA alleles, the disclosed systems extract a genomic sample's reads corresponding to an HLA genomic region and align the extracted reads with HLAallele-reference sequences. The disclosed systems further select a subset of read alignments for the extracted reads based on alignment scores for alignments between the extracted reads and the HLAallele-reference sequences. Based on the selected subset of read alignments, the disclosed systems group individual reads into HLA equivalence classes and determine candidate HLA alleles for the genome sample at one or more HLA loci. From among the candidate HLA alleles, the disclosed systems determine genotype calls that a genomic sample includes particular HLA alleles at one or more HLA loci.

## Description

### CROSS-REFERENCE TO RELATED APPLICATIONS

The present application claims the benefit of, and priority to, U.S. Provisional Application No. 63/367,107, entitled "IMPROVED HUMAN LEUKOCYTE ANTIGEN (HLA) GENOTYPING," filed on June 27, 2022. The aforementioned application is hereby incorporated by reference in its entirety.

### BACKGROUND

In recent years, biotechnology firms and research institutions have improved hardware and software for sequencing nucleotides and determining nucleobase calls for genomic samples. For instance, some existing sequencing machines and sequencing-data-analysis software (together "existing sequencing systems") predict individual nucleobases within sequences by using conventional Sanger sequencing or sequencing-by-synthesis (SBS) methods. When using SBS, existing sequencing systems can monitor many thousands of oligonucleotides being synthesized in parallel from templates to predict nucleobase calls for growing nucleotide reads. In many existing sequencing systems, a camera captures images of irradiated fluorescent tags incorporated into oligonucleotides. After capturing such images, some existing sequencing systems determine nucleobase calls for nucleotide reads corresponding to the oligonucleotides and send base-call data to a computing device with sequencing-data-analysis software, which aligns nucleotide reads with a reference genome. Based on differences between the aligned nucleotide reads and the reference genome, existing systems can further utilize a variant caller to identify variants of a genomic sample, such as single nucleotide polymorphisms (SNPs), and/or a human leukocyte antigen (HLA) caller to determine HLA alleles for the genomic sample.

In some cases, existing sequencing systems use specialized software applications to determine HLA alleles because of the importance of the HLA genomic region as a biomarker. The HLA complex represents the human version of the major histocompatibility complex and a series of genes on chromosome 6 in humans that encodes for cell-surface proteins that help regulate humans' immune systems. Various genes within the HLA complex serve as important biomarkers for cancer, autoimmune diseases (e.g., diabetes), celiac disease, and other diseases. The HLA complex is generally divided into three classes in which HLA-I generally encode peptides inside a cell, HLA-II encodes antigens outside a cell, and HLA-III encodes components of a complementary system.

In addition to representing critical biomarkers, the HLA complex includes a high degree of HLA-allele variation among populations that has historically challenged the accuracy of existing sequencing system's HLA genotyping. For instance, researchers have identified at least 22,000 different HLA alleles among humans, including at least 13,000 different HLA-encoded proteins. Indeed, some HLA alleles differ by a single nucleobase. To further complicate HLA-allele variation, many HLA genes exhibit a high degree of homology with HLA pseudogenes that represent non-functional and imperfect copies of HLA genes. Indeed, in part because of the high degree of variation and homology, existing sequencing systems typically determine genotype calls for HLA alleles at two-field resolution.

Despite the biological importance of-and because of the high degree of variation in-the HLA complex, existing sequencing systems employ various different assays to determine HLA alleles for genomic samples with varying and imperfect accuracy. For example, some existing sequencing systems analyze nucleotide reads corresponding to the HLA complex as part of whole genome sequencing (WGS) to determine HLA alleles with (i) approximately 88-89% accuracy in comparison to HISAT2 genotypes as ground truths using a tumor analysis mode and (ii) approximately 91% accuracy in comparison to Sanger sequencing of exons as ground truths. As a further example, some existing sequencing systems analyze such HLA-corresponding reads as part of whole exome sequencing (WES) to determine HLA alleles with approximately 93-94% accuracy in comparison to histogenetics as ground truths. By contrast, existing sequencing systems analyze such HLA-corresponding reads as part of tumor gene panels, such as TruSight^{®} Oncology, to determine HLA alleles with approximately 90-91% accuracy in comparison to HISAT2 genotypes or International Histocompatibility Working Group (IHWG) sequences as ground truths. Despite accuracies between approximately 88% and 94%, a single error in HLA genotype call in a clinical setting can have significant consequences in detecting various diseases.

To compensate for the limited accuracy of existing assays that determine HLA alleles, some existing sequencing systems can perform a combination of different assays on a genomic sample to increase confidence in HLA genotyping for a genomic sample. For instance, some existing sequencing systems may run two or more assays for WGS, WES, or tumor-gene panels. By running a combination of assays-and sometimes using different specialized sequencing devices-existing sequencing systems multiply the computer processing and time to determine accurate HLA alleles for a genomic sample.

These, along with additional problems and issues exist in existing sequencing systems.

### SUMMARY

This disclosure describes one or more embodiments of systems, methods, and non-transitory computer readable storage media that solve one or more of the problems described above or provide other advantages over the art. In particular, the disclosed systems can accurately genotype one or more human leukocyte antigen (HLA) alleles from a genomic sample by using alignment-score-based filtering and read-support-equivalence grouping of reads for genotype inference. To genotype HLA alleles, the disclosed systems extract a genomic sample's reads corresponding to an HLA genomic region and align the extracted reads with HLA-allele-reference sequences. The disclosed systems further select a subset of read alignments for the extracted reads based on alignment scores for alignments between the extracted reads and the HLA-allele-reference sequences. Based on the selected subset of read alignments, the disclosed systems group individual reads into HLA equivalence classes and determine candidate HLA alleles for the genome sample at one or more HLA loci. From among the candidate HLA alleles, the disclosed systems determine genotype calls that a genomic sample includes particular HLA alleles at one or more HLA loci.

Additional features and advantages of one or more embodiments of the present disclosure will be set forth in the description which follows, and in part will be obvious from the description, or may be learned by the practice of such example embodiments.

### BRIEF DESCRIPTION OF THE DRAWINGS

The detailed description refers to the drawings briefly described below.

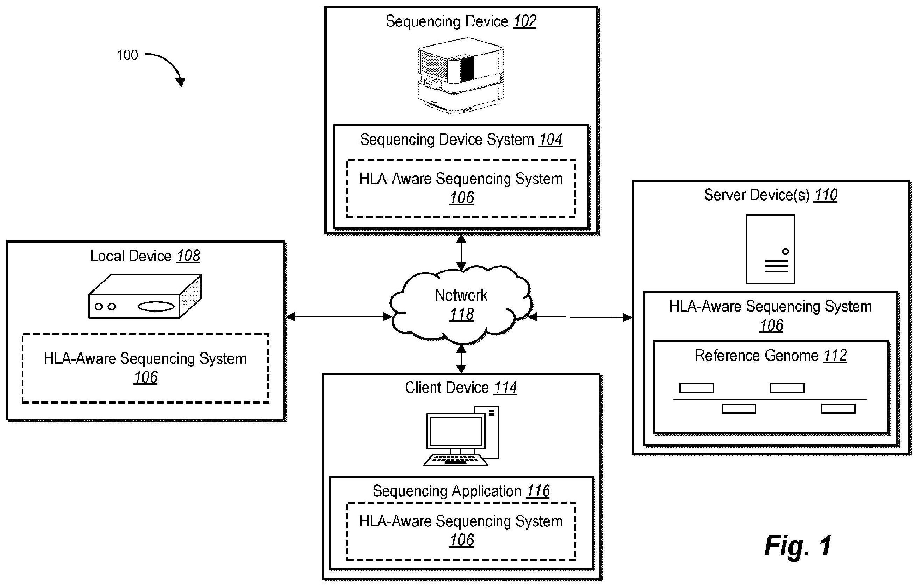

FIG. 1 illustrates an environment in which an HLA-aware sequencing system can operate in accordance with one or more embodiments of the present disclosure.

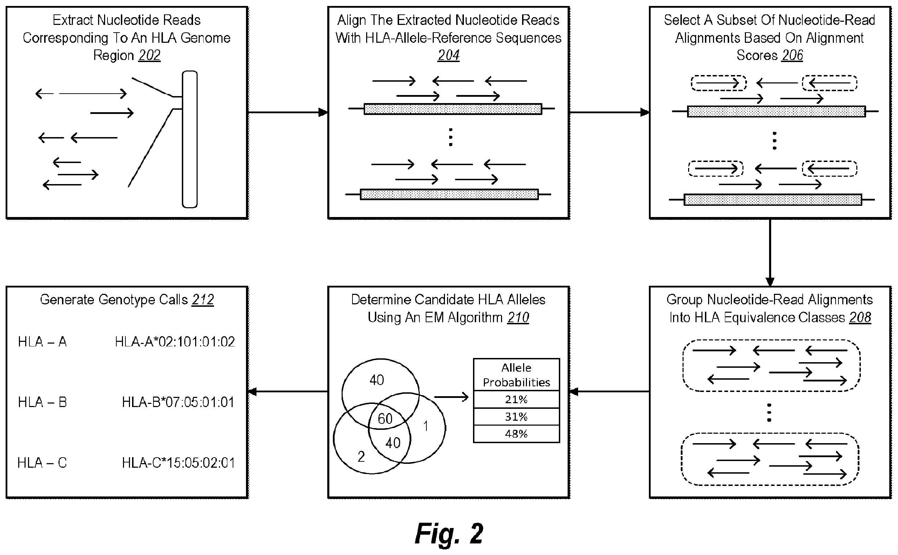

FIG. 2 illustrates the HLA-aware sequencing system determining a genotype of one or more HLA alleles from a genomic sample by filtering nucleotide reads corresponding to an HLA region according to scores for alignments with HLA-allele-reference sequences and grouping the filtered nucleotide reads into HLA equivalence classes for statistical inference in accordance with one or more embodiments of the present disclosure.

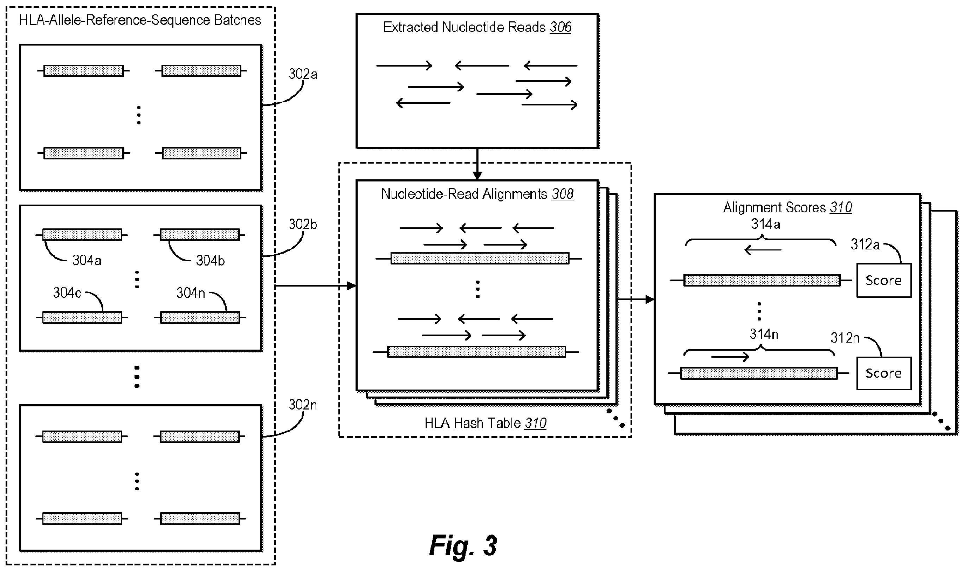

FIG. 3 illustrates the HLA-aware sequencing system grouping HLA-allele-reference sequences into batches, aligning extracted nucleotide reads with the HLA-allele-reference sequences in the batches, and determining alignment scores for each nucleotide-read alignment in accordance with one or more embodiments of the present disclosure.

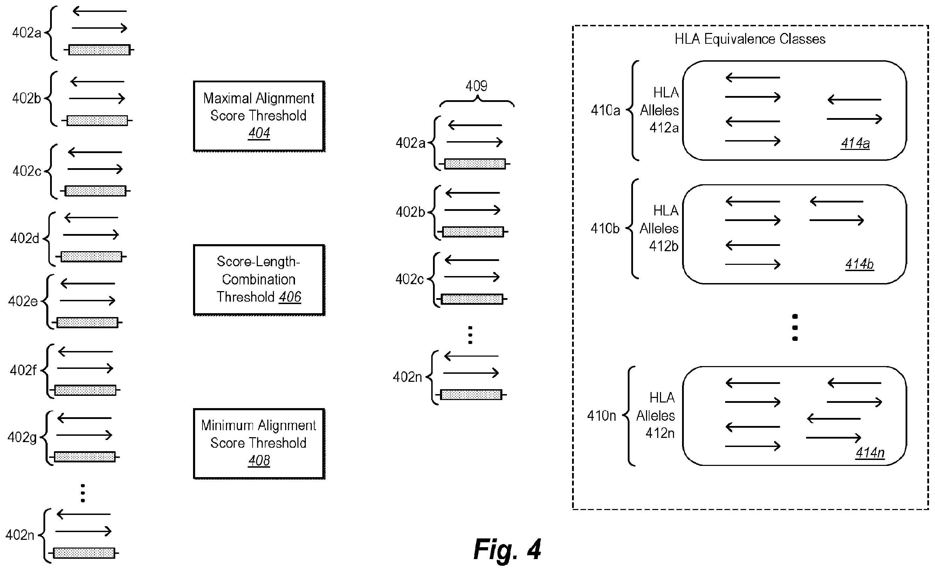

FIG. 4 illustrates the HLA-aware sequencing system selecting a subset of nucleotide-read alignments based on various alignment-score thresholds and grouping the selected subset of nucleotide-read alignments into HLA equivalence classes in accordance with one or more embodiments of the present disclosure.

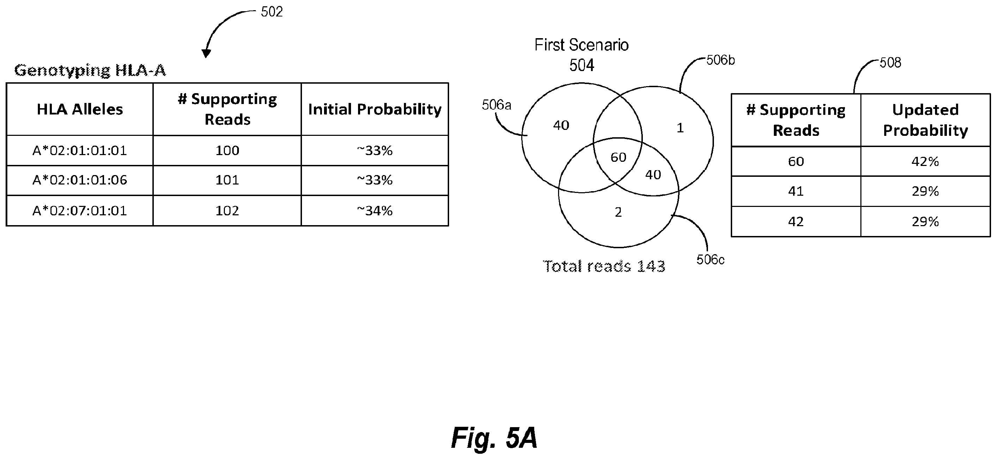

FIGS. 5A-5C illustrate the HLA-aware sequencing system performing an expectation-maximization (EM) algorithm to determine posterior candidate allele probabilities that a genomic sample comprises candidate HLA alleles based on nucleotide reads grouped into HLA equivalence classes in accordance with one or more embodiments of the present disclosure.

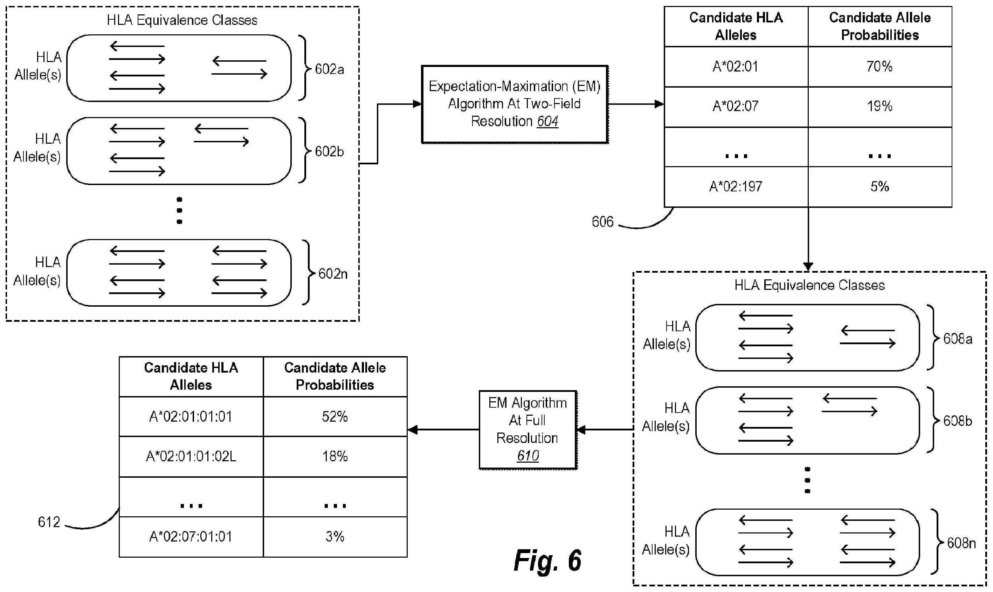

FIG. 6 illustrates the HLA-aware sequencing system performing a two-stage EM algorithm to determine candidate allele probabilities at different HLA allele resolutions in accordance with one or more embodiments of the present disclosure.

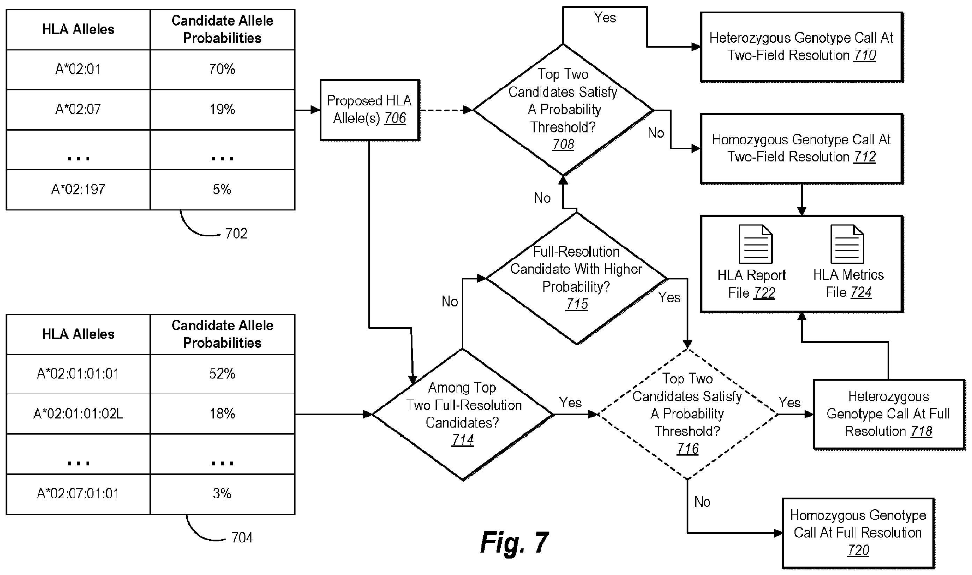

FIG. 7 illustrates the HLA-aware sequencing system generating genotype calls that a genomic sample comprises one or more HLA alleles at an HLA locus at either two-field resolution or full resolution in accordance with one or more embodiments of the present disclosure.

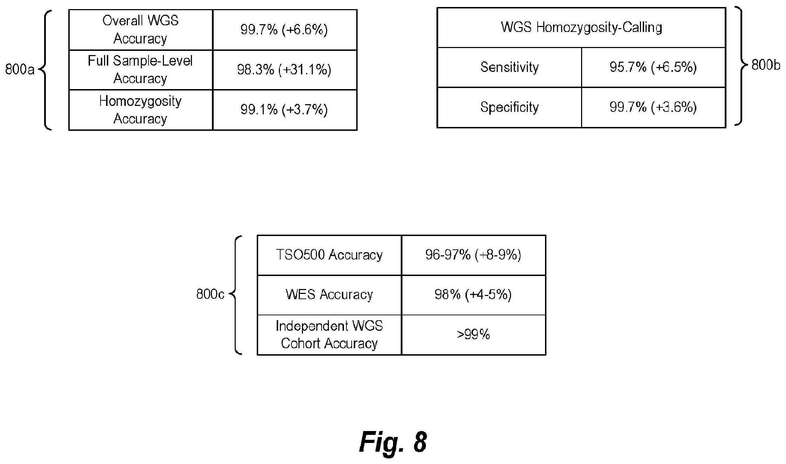

FIG. 8 illustrates tables showing an accuracy, sensitivity, or specificity with which the HLA-aware sequencing system generates HLA genotype calls for whole genome sequencing (WGS), whole exome sequencing (WES), and tumor gene panels in accordance with one or more embodiments of the present disclosure.

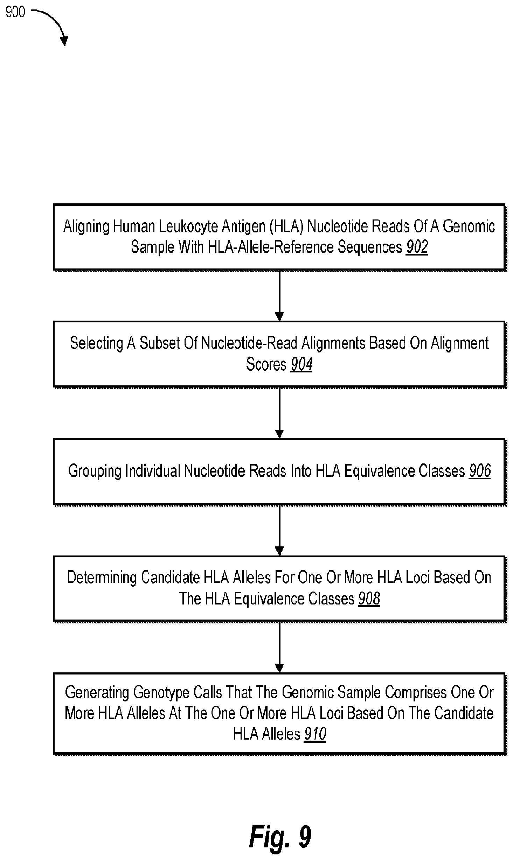

FIG. 9 illustrate series of acts for genotyping one or more human leukocyte antigen (HLA) alleles from a genomic sample by using alignment-score-based filtering and read-support-equivalence grouping of nucleotide reads for genotype inference in accordance with one or more embodiments of the present disclosure.

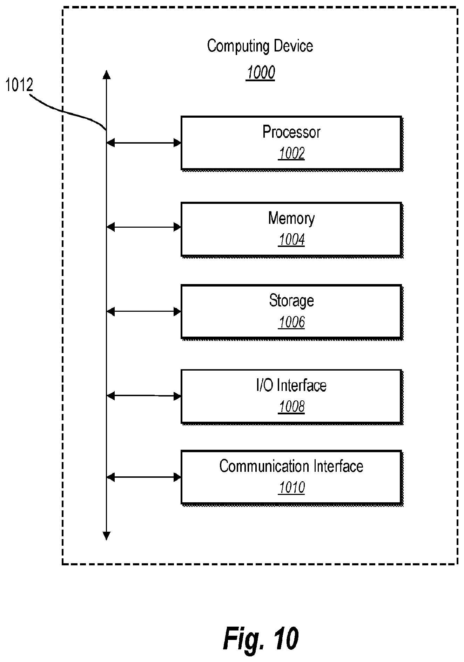

FIG. 10 illustrates a block diagram of an example computing device in accordance with one or more embodiments of the present disclosure.

### DETAILED DESCRIPTION

This disclosure describes one or more embodiments of a human-leukocyte-antigen-aware (or "HLA-aware") sequencing system that can accurately determine a genotype of one or more human leukocyte antigen (HLA) alleles from a genomic sample by filtering nucleotide reads corresponding to an HLA region according to scores for alignments with HLA-allele-reference sequences and grouping the filtered nucleotide reads into HLA equivalence classes for statistical inference. To genotype HLA alleles, the HLA-aware sequencing system extracts a genomic sample's nucleotide reads corresponding to an HLA genomic region and aligns the extracted nucleotide reads with HLA-allele-reference sequences from an HLA-sequence database. The HLA-aware sequencing system further selects a subset of read alignments for the extracted nucleotide reads based on alignment scores for alignments between the extracted nucleotide reads and the HLA-allele-reference sequences. Based on the selected subset of read alignments, the HLA-aware sequencing system groups individual nucleotide reads into HLA equivalence classes and determines candidate HLA alleles for the genome sample at one or more HLA loci by running an expectation-maximation (EM) algorithm on the nucleotide reads grouped by the HLA equivalence classes. From among the candidate HLA alleles, the HLA-aware sequencing system determines genotype calls that a genomic sample includes particular HLA alleles at one or more HLA loci (e.g., HLA-A, HLA-B, and HLA-C loci).

As just noted, the HLA-aware sequencing system can extract nucleotide reads of a genomic sample that correspond to an HLA genomic region. In some embodiments, for instance, the HLA-aware sequencing system aligns a set of nucleotide reads from a genomic sample with a reference genome. The HLA-aware sequencing system subsequently identifies a subset of nucleotide reads that align or overlap with the HLA genomic region. Such an HLA genomic region comprises one or more HLA genes can be identified by genomic coordinates-including a threshold flanking region adjacent to HLA genes.

To align the extracted reads with HLA-allele-reference sequences, in some embodiments, the HLA-aware sequencing system groups HLA-allele-reference sequences into batches and align the extracted reads-batch by batch-with the HLA-allele-reference sequences. While existing sequencing systems for whole genome sequencing (WGS) frequently limit the number of alternate reference sequences used for alignment nucleotide reads, the HLA-aware sequencing system avoids or removes such limitations by using a batching approach. In some embodiments, for instance, the HLA-aware sequencing system groups nearly 8,000 HLA-allele-reference sequences into approximately 16 batches of approximately 500 HLA allele-references each and aligns each extracted nucleotide read with each of the HLA-allele-reference sequence.

After determining HLA-specific alignments for the extracted reads, as suggested above, the HLA-aware sequencing system determines alignment scores for candidate alignments between extracted nucleotide reads and HLA-allele-reference sequence. The HLA-aware sequencing system further selects a subset of read alignments in which the nucleotide read exhibits a highest alignment score, such as when each mate of a paired-end read exhibits a maximal alignment score. From among the selected subset of read alignments, the HLA-aware sequencing system removes read alignments that have an alignment score that does not satisfy one or more criteria: (i) a score-length-combination threshold based on a nucleobase length of a corresponding nucleotide read and (ii) a minimum alignment score threshold.

Having removed nucleotide reads that fail certain thresholds, in some embodiments, the HLA-aware sequencing system groups the filtered subset of nucleotide reads into HLA equivalence classes. Such HLA equivalence classes represent allele groups that are equally supported by groups of filtered nucleotide reads. The HLA-aware sequencing system subsequently performs an expectation-maximization (EM) algorithm on individual nucleotide reads grouped according to the HLA equivalence classes to determine candidate alleles for each HLA locus.

Unlike existing methods of HLA genotyping, the HLA-aware sequencing system can run a two-stage EM algorithm to improve the accuracy of HLA genotyping. In a first EM algorithm stage, for instance, the HLA-aware sequencing system determines a first set of candidate allele probabilities that a genomic sample includes candidate HLA alleles at two-field resolution by analyzing individual nucleotide reads grouped by the HLA equivalence classes. In a second EM algorithm stage, the HLA-aware sequencing system determines a second set of candidate allele probabilities that the genomic sample includes candidate HLA alleles at full resolution by analyzing nucleotide reads regrouped into HLA equivalence classes based on the candidate HLA alleles determined from the first EM algorithm stage.

Based on the candidate HLA alleles, the HLA-aware sequencing system determines genotype calls that a genomic sample comprises HLA alleles at one or more HLA loci. The HLA-aware sequencing system can genotype HLA alleles at one or both of two-field resolution and full resolution. If, for instance, a proposed HLA allele at two-field resolution is among a top two candidate HLA alleles at full resolution that exhibit the highest candidate allele probabilities (e.g., top two posterior candidate allele probabilities) from a second stage of the EM algorithm, the HLA-aware sequencing system determines a genotype call of the proposed HLA allele at full resolution. If, by contrast, a proposed HLA allele at two-field resolution is not among the top two candidate HLA alleles at full resolution, the HLA-aware sequencing system either (i) determines a genotype call of the proposed HLA allele at two-field resolution when no candidate HLA allele at full resolution exhibits a higher candidate allele probability than the proposed HLA allele or (ii) determines a genotype call of a candidate HLA allele at full resolution when the candidate HLA allele at full resolution exhibits a higher candidate allele probability than the proposed HLA allele.

As indicated above, the HLA-aware sequencing system provides several technical advantages relative to existing sequencing systems by, for example, improving genotyping accuracy, genotyping specificity, and computational efficiency relative to existing sequencing systems. For example, the HLA-aware sequencing system improves the accuracy of HLA genotyping by selecting a subset of nucleotide reads according to alignment scores with HLA-allele-reference sequences. Unlike existing sequencing systems that determine HLA alleles from a general set of nucleotide reads corresponding to an HLA genomic region, the HLA-aware sequencing system can intelligently filter and group HLA-corresponding nucleotide reads. By filtering reads corresponding to an HLA genomic region according to alignments scores for read alignments with HLA-allele-reference sequences and grouping the filtered reads into HLA equivalence classes, in some cases, the HLA-aware sequencing system identifies a more reliable subset of nucleotide reads from a genomic sample upon which to determine HLA genotypes. By further performing a unique two-stage EM algorithm, in some cases, the HLA-aware sequencing system leverages HLA equivalence groups to intelligently identify the candidate HLA alleles probabilistically supported by a filtered subset of nucleotide reads corresponding to an HLA genomic region.

Indeed, as explained below, the HLA-aware sequencing system improves HLA genotyping accuracy in several measures. While existing sequencing systems determine HLA alleles with approximately 88%-94% accuracy, the HLA-aware sequencing system determines HLA alleles with approximately 96%-99%. Further to the point, the HLA-aware sequencing system also improves HLA genotyping accuracy across different assays, including improved HLA genotyping accuracy in each of WGS, WES, and tumor gene panels.

In addition to improving HLA genotyping accuracy, the HLA-aware sequencing system improves the specificity with which genotype calls for HLA alleles are generated. As noted above, existing sequencing systems typically determine genotype calls for HLA alleles at two-field resolution. By contrast, the HLA-aware sequencing system can determine genotype calls for HLA alleles at full resolution. As explained further below, in some embodiments, the HLA-aware sequencing system determines candidate HLA alleles at full resolution using a unique combination EM algorithm. The HLA-aware sequencing system can further use such full-resolution candidate HLA alleles as a reference point (or override) for two-resolution candidate HLA alleles from which genotype calls are selected or further fleshed out into full resolution.

Beyond improved genotyping accuracy and specificity, in some embodiments, the HLA-aware sequencing system improves computational efficiency by reducing the number of sequencing assays and computational devices used to determine accurate genotype calls for HLA alleles. As noted above, some existing sequencing systems consume significant computer processing and time by running run two or more assays for WGS, WES, or tumor-gene panels to determine HLA alleles, where some such assays require different specialized sequencing devices. By comparing the nucleotide reads to a reference genome for WGS, reference exomes for WES, and reference genes for a gene panel, existing sequencing systems can increase confidence of an HLA genotype call from one assay with another assay. In contrast to such existing sequencing systems, in some embodiments, the HLA-aware sequencing system facilitates a more computationally efficient approach by using a specialized sequencing device to determine HLA genotype calls-without or with fewer additional assays or specialized sequencing devices-to determine HLA genotype calls for a genomic sample. Accordingly, the HLA-aware sequencing system can obviate some or all extra assays for HLA genotyping.

As illustrated by the foregoing discussion, the present disclosure utilizes a variety of terms to describe features and advantages of the HLA-aware sequencing system. As used herein, for example, the term "HLA allele" refers to a version or alternative form of an HLA gene or HLA nucleotide sequence. In some cases, an HLA allele is represented as a digital nucleotide sequence or at various different resolutions. For instance, an HLA allele may be represented by an encoded nucleotide sequence, such as by single-letter codes representing individual nucleobases (e.g., A, C, T, G), corresponding to particular genomic coordinates or HLA locus. Additionally or alternatively, an HLA allele may be represented by a combination of prefixes and fields, such as the combination of prefixes and fields used by the World Health Organization (WHO) Nomenclature Committee for Factors of the HLA System.

To illustrate, an HLA allele may be represented by one or more of an HLA prefix, a gene, a separator, a first field for allele group, a second field for a specific HLA protein, a third field for a number representing a synonymous DNA substitution within a coding region, a fourth field for a number representing differences in a non-coding region, and a suffix to indicate changes in expression. For example, an HLA allele may be represented at full resolution as "HLA-A*02:101:01:02N" or "HLA-A*02:101:01:02," where "HLA" constitutes the HLA prefix, "A" designates an HLA gene, "*" represents a separator, "02" is a value in the first field for allele group, "101" is a value in the second field for a specific HLA protein, "01" is a value in the third field representing a synonymous DNA substitution in a coding region, "02" is a value in the fourth field representing differences in a non-coding region, and "N" is a suffix indicating changes in expression.

Relatedly, the term "candidate HLA allele" refers to a potential or proposed HLA allele selected or identified for a genomic sample based on nucleotide reads from the genomic sample corresponding to an HLA genomic region. In particular, a candidate HLA allele includes a potential or proposed HLA allele selected by a model for a particular locus of a genomic sample based on allele probabilities determined by the model from nucleotide reads from the genomic sample corresponding to an HLA genomic region. As suggested above, in some embodiments, the HLA-aware sequencing system use an expectation-maximization (EM) algorithm to determine candidate HLA allele probabilities that a genomic sample comprises particular candidate HLA alleles.

As further used herein, the term "two-field resolution" refers to a resolution level of two fields that identifies an HLA allele. In particular, two-field resolution (i) includes an identification of an HLA allele with a first field for an allele group for HLA and a second field for a specific HLA protein but (ii) excludes a third field for a number representing a synonymous DNA substitution and a fourth field for a number representing differences in a non-coding region. Critically, two-field resolution can include prefixes and separators. For example, a particular candidate HLA allele or a genotype call of A particular HLA allele can be represented as "HLA-A*02:101."

By contrast, the term "full resolution" (or "four-field resolution") refers to a resolution level of four fields that identifies an HLA allele. In particular, full resolution includes an identification of an HLA allele with a first field for an allele group for HLA, a second field for a specific HLA protein, a third field for a number representing a synonymous DNA substitution, and a fourth field for a number representing differences in a non-coding region. According, in some cases, full resolution of an HLA allele captures intron variants under the nomenclature of the WHO Nomenclature Committee for Factors of the HLA System. Critically, four-field resolution can include prefixes and separators. For example, a particular candidate HLA allele or a genotype call of A particular HLA allele can be represented as HLA-A*02:101:01:02N" or "HLA-A*02:101:01:02."

Relatedly, the term "HLA-allele-reference sequence" refers to a digital representation of a nucleotide sequence for a recognized or known HLA allele. In particular, an HLA-allele-reference sequence includes a digital representation of a full nucleotide sequence for a named HLA allele from an HLA-sequence database. In some embodiments, for instance, HLA-allele-reference sequences represent officially named HLA alleles that have digitally encoded sequences accessible for download or identification from the Immuno Polymorphism Database-ImMunoGeneTics project human leukocyte antigen (IPD-IMGT/HLA) database.

As further used herein, the term "HLA equivalence class" refers to a group of one or more HLA alleles that are supported by a group of nucleotide reads. In particular, an HLA equivalence class include a group of HLA alleles that a group of paired-end nucleotide reads from a genomic sample equally support in terms of alignment. For example, an HLA equivalence class may include a first HLA allele, a second HLA allele, and a third HLA allele at two-field or full resolution with which a group of nucleotide reads equally aligns or matches.

As suggested above, a "locus" refers to a physical site or location within a genome, such as a site or location of a gene or another DNA segment. In particular, a locus includes a set of genomic coordinates that identifies a site or location of a gene or another DNA segment on a chromosome. Accordingly, an HLA locus represents a site or location of an HLA gene (or other DNA segment from an HLA genomic region) within a genome. In some cases, an HLA locus can include a site or location of a particular HLA gene, such as HLA-A, HLA-B, or HLA-C. While this disclosure frequently uses examples of HLA genes from HLA class 1, in some embodiments, the HLA-aware sequencing system likewise determines genotype calls that a genomic sample comprises an HLA allele at other HLA loci, such as HLA class II genes (e.g., HLA-DR, HLA-DQ, HLA-DP).

As also used herein, the term "reference genome" refers to a digital nucleic acid sequence assembled as a representative example (or representative examples) of genes and other genetic sequences of an organism. Regardless of the sequence length, in some cases, a reference genome represents an example set of genes or a set of nucleic acid sequences in a digital nucleic acid sequence determined as representative of an organism. For example, a linear human reference genome may be GRCh38 (or other versions of reference genomes) from the Genome Reference Consortium.

Additionally, as used herein, the term "genomic coordinate" refers to a particular location or position of a nucleotide base within a genome (e.g., an organism's genome or a reference genome). In some cases, a genomic coordinate includes an identifier for a particular chromosome of a genome and an identifier for a position of a nucleotide base within the particular chromosome. For instance, a genomic coordinate or coordinates may include a number, name, or other identifier for a chromosome (e.g., chr1 or chrX) and a particular position or positions, such as numbered positions following the identifier for a chromosome (e.g., chr1:1234570 or chr1:1234570-1234870). Further, in certain implementations, a genomic coordinate refers to a source of a reference genome (e.g., mt for a mitochondrial DNA reference genome or SARS-CoV-2 for a reference genome for the SARS-CoV-2 virus) and a position of a nucleotide-base within the source for the reference genome (e.g., mt:16568 or SARS-CoV-2:29001). By contrast, in certain cases, a genomic coordinate refers to a position of a nucleotide-base within a reference genome without reference to a chromosome or source (e.g., 29727).

As used herein, a "genomic region" refers to a range of genomic coordinates. Like genomic coordinates, in certain implementations, a genomic region may be identified by an identifier for a chromosome and a particular position or positions, such as numbered positions following the identifier for a chromosome (e.g., chr1:1234570-1234870). In various implementations, a genomic coordinate includes a position within a reference genome. In some cases, a genomic coordinate is specific to a particular reference genome.

Relatedly, the term "HLA genomic region" refers to a genomic region that includes HLA genes and surrounding or flanking nucleotide sequences. In particular, an HLA genomic region includes an HLA MHC complex and surrounding or flunking nucleotide sequences within a threshold number of nucleobases. Such a threshold number of nucleobases may, for instance, be 1,000 nucleobase on each side of the HLA MHC complex.

Also, as used herein, the term "genomic sample" refers to a target genome or portion of a genome undergoing sequencing. For example, a sample genome includes a sequence of nucleotides isolated or extracted from a sample organism (or a copy of such an isolated or extracted sequence). In particular, a sample genome includes a full genome that is isolated or extracted (in whole or in part) from a sample organism and composed of nitrogenous heterocyclic bases. A sample genome can include a segment of deoxyribonucleic acid (DNA), ribonucleic acid (RNA), or other polymeric forms of nucleic acids or chimeric or hybrid forms of nucleic acids noted below. In some cases, the sample genome is found in a sample prepared or isolated by a kit and received by a sequencing device.

As further used herein, the term "nucleotide read" (or simply "read") refers to an inferred sequence of one or more nucleobases (or nucleobase pairs) from all or part of a sample nucleotide sequence (e.g., a sample genomic sequence, cDNA). In particular, a nucleotide read includes a determined or predicted sequence of nucleobase calls for a nucleotide sequence (or group of monoclonal nucleotide sequences) from a sample library fragment corresponding to a genome sample. For example, in some cases, a sequencing device determines a nucleotide read by generating nucleobase calls for nucleobases passed through a nanopore of a nucleotide-sample slide, determined via fluorescent tagging, or determined from a cluster in a flow cell.

As further used herein, the term "alignment score" refers to a numeric score, metric, or other quantitative measurement evaluating an accuracy of an alignment between a nucleotide read (or a fragment of the nucleotide read) and another nucleotide sequence. In particular, an alignment score includes a metric indicating a degree to which the nucleobases of a nucleotide read match or are similar to (i) an HLA-allele-reference sequence or (ii) a reference sequence or an alternate contiguous sequence from a reference genome. In certain implementations, an alignment score takes the form of a Smith-Waterman score or a variation or version of a Smith-Waterman score for local alignment, such as various settings or configurations used by DRAGEN by Illumina, Inc. for Smith-Waterman scoring.

As further used herein, the term "genotype call" refers to a determination or prediction of a particular genotype of a genomic sample at a genomic locus. In particular, a genotype call can include a prediction of a particular genotype of a genomic sample with respect to a reference genome or a reference sequence (e.g., HLA-allele-reference sequence) at a genomic coordinate or a genomic region. A genotype call is often determined for a genomic coordinate or genomic region at which an SNP or other variant has been identified for a population of organisms. In this disclosure, among other genotype calls, the HLA-aware sequencing system generates genotype calls that a genomic sample comprises HLA allele at HLA loci.

The following paragraphs describe the HLA-aware sequencing system with respect to illustrative figures that portray example embodiments and implementations. For example, FIG. 1 illustrates a schematic diagram of a computing system 100 in which an HLA-aware sequencing system 106 operates in accordance with one or more embodiments. As illustrated, the computing system 100 includes a sequencing device 102 connected to a local device 108 (e.g., a local server device), one or more server device(s) 110, and a client device 114. As shown in FIG. 1, the sequencing device 102, the local device 108, the server device(s) 110, and the client device 114 can communicate with each other via a network 118. The network 118 comprises any suitable network over which computing devices can communicate. Example networks are discussed in additional detail below with respect to FIG. 10. While FIG. 1 shows an embodiment of the HLA-aware sequencing system 106, this disclosure describes alternative embodiments and configurations below.

As indicated by FIG. 1, the sequencing device 102 comprises a computing device and a sequencing device system 104 for sequencing a genomic sample or other nucleic-acid polymer. In some embodiments, by executing the sequencing device system 104 using a processor, the sequencing device 102 analyzes nucleotide fragments or oligonucleotides extracted from genomic samples to generate nucleotide reads or other data utilizing computer implemented methods and systems either directly or indirectly on the sequencing device 102. More particularly, the sequencing device 102 receives nucleotide-sample slides (e.g., flow cells) comprising nucleotide fragments extracted from samples and further copies and determines the nucleobase sequence of such extracted nucleotide fragments.

In one or more embodiments, the sequencing device 102 utilizes SBS to sequence nucleotide fragments into nucleotide reads and determine nucleobase calls for the nucleotide reads. In addition or in the alternative to communicating across the network 118, in some embodiments, the sequencing device 102 bypasses the network 118 and communicates directly with the local device 108 or the client device 114. By executing the sequencing device system 104, the sequencing device 102 can further store the nucleobase calls as part of base-call data that is formatted as a binary base call (BCL) file and send the BCL file to the local device 108 and/or the server device(s) 110.

As further indicated by FIG. 1, the local device 108 is located at or near a same physical location of the sequencing device 102. Indeed, in some embodiments, the local device 108 and the sequencing device 102 are integrated into a same computing device. The local device 108 may run the HLA-aware sequencing system 106 to generate, receive, analyze, store, and transmit digital data, such as by receiving base-call data or determining variant calls based on analyzing such base-call data. As shown in FIG. 1, the sequencing device 102 may send (and the local device 108 may receive) base-call data generated during a sequencing run of the sequencing device 102. By executing software in the form of the HLA-aware sequencing system 106, the local device 108 may align nucleotide reads with a reference genome 112 and determine genetic variants based on the aligned nucleotide reads. The local device 108 may also communicate with the client device 114. In particular, the local device 108 can send data to the client device 114, including a variant call file (VCF), an HLA report file, an HLA metrics file, or other information indicating nucleobase calls, sequencing metrics, error data, or other metrics.

As further indicated by FIG. 1, the server device(s) 110 are located remotely from the local device 108 and the sequencing device 102. Similar to the local device 108, in some embodiments, the server device(s) 110 include a version of the HLA-aware sequencing system 106. Accordingly, the server device(s) 110 may generate, receive, analyze, store, and transmit digital data, such as by receiving base-call data or determining variant calls or genotype calls for HLA alleles based on analyzing such base-call data. As indicated above, the sequencing device 102 may send (and the server device(s) 110 may receive) base-call data from the sequencing device 102. The server device(s) 110 may also communicate with the client device 114. In particular, the server device(s) 110 can send data to the client device 114, including VCFs, HLA report files, HLA metrics files, or other sequencing related information.

In some embodiments, the server device(s) 110 comprise a distributed collection of servers where the server device(s) 110 include a number of server devices distributed across the network 118 and located in the same or different physical locations. Further, the server device(s) 110 can comprise a content server, an application server, a communication server, a web-hosting server, or another type of server.

As indicated above, as part of the server device(s) 110 or the local device 108, the HLA-aware sequencing system 106 can accurately genotype one or more HLA alleles from a genomic sample by filtering reads corresponding to an HLA region according to alignments scores for read alignments with HLA-allele-reference sequences and grouping the filtered reads into HLA equivalence classes for statistical inference. For instance, the HLA-aware sequencing system 106 can extract nucleotide reads corresponding to an HLA genomic region of a genomic sample and align the extracted nucleotide reads with HLA-allele-reference sequences. The HLA-aware sequencing system 106 further selects a subset of read alignments for the extracted nucleotide reads based on alignment scores for alignments between the extracted nucleotide reads and the HLA-allele-reference sequences. Based on the selected subset of read alignments, the HLA-aware sequencing system 106 groups individual nucleotide reads into HLA equivalence classes and determines candidate HLA alleles for the genome sample at one or more HLA loci. From among the candidate HLA alleles, the HLA-aware sequencing system 106 determines genotype calls that a genomic sample includes particular HLA alleles at one or more HLA loci.

As further illustrated and indicated in FIG. 1, by executing a sequencing application 116, the client device 114 can generate, store, receive, and send digital data. In particular, the client device 114 can receive sequencing data from the local device 108 or receive call files (e.g., BCL) and sequencing metrics from the sequencing device 102. Furthermore, the client device 114 may communicate with the local device 108 or the server device(s) 110 to receive a VCF, HLA report file, or HLA metrics file comprising nucleobase calls, genotype calls, and/or other metrics, such as a base-call-quality metrics or pass-filter metrics. The client device 114 can accordingly present or display information pertaining to genotype calls, variant calls, or other nucleobase calls within a graphical user interface of the sequencing application 116 to a user associated with the client device 114. For example, the client device 114 can present genotype calls for HLA alleles and/or sequencing metrics for a sequenced genomic sample within a graphical user interface of the sequencing application 116.

Although FIG. 1 depicts the client device 114 as a desktop or laptop computer, the client device 114 may comprise various types of client devices. For example, in some embodiments, the client device 114 includes non-mobile devices, such as desktop computers or servers, or other types of client devices. In yet other embodiments, the client device 114 includes mobile devices, such as laptops, tablets, mobile telephones, or smartphones. Additional details regarding the client device 114 are discussed below with respect to FIG. 10.

As further illustrated in FIG. 1, the client device 114 includes the sequencing application 116. The sequencing application 116 may be a web application or a native application stored and executed on the client device 114 (e.g., a mobile application, desktop application). The sequencing application 116 can include instructions that (when executed) cause the client device 114 to receive data from the HLA-aware sequencing system 106 and present, for display at the client device 114, base-call data or data from a VCF, HLA report file, or HLA metrics file.

As further illustrated in FIG. 1, a version of the HLA-aware sequencing system 106 may be located and implemented (e.g., entirely or in part) on the client device 114 or the sequencing device 102. In yet other embodiments, the HLA-aware sequencing system 106 is implemented by one or more other components of the computing system 100, such as the local device 108. In particular, the HLA-aware sequencing system 106 can be implemented in a variety of different ways across the sequencing device 102, the local device 108, the server device(s) 110, and the client device 114. For example, the HLA-aware sequencing system 106 can be downloaded from the server device(s) 110 to the HLA-aware sequencing system 106 and/or the local device 108 where all or part of the functionality of the HLA-aware sequencing system 106 is performed at each respective device within the computing system 100.

As indicated above, the HLA-aware sequencing system 106 can genotype one or more HLA alleles from a genomic sample by using alignment-score-based filtering and read-support-equivalence grouping of nucleotide reads for genotype inference. FIG. 2 depicts an overview of such embodiments for the HLA-aware sequencing system 106. In accordance with one or more embodiments, FIG. 2 illustrates an example of the HLA-aware sequencing system 106 determining genotype calls that a genomic sample comprises particular HLA alleles at various loci by filtering a subset of HLA-corresponding nucleotide reads from the genomic sample according to scores for alignments between such reads with HLA-allele-reference sequences, grouping the filtered subset of nucleotide reads into HLA equivalence classes, and performing an EM algorithm on the HLA-equivalence-classed nucleotide reads.

As shown in FIG. 2, the HLA-aware sequencing system 106 extracts nucleotide reads corresponding to an HLA genomic region 202. For example, in some embodiments, a sequencing device sends (and the HLA-aware sequencing system 106 receives) base-call data comprising digital representations of a set of nucleotide reads from a genomic sample. The HLA-aware sequencing system 106 further aligns the set of nucleotide reads with a reference genome. Based on these alignments, in some embodiments, the HLA-aware sequencing system 106 subsequently identifies a subset of nucleotide reads that align or overlap with the HLA MHC complex-including individual nucleotide reads that align with HLA genes within the HLA MHC complex and individual nucleotide reads that align with genomic regions within a threshold number of nucleobases from either end of the HLA genes.

As further indicated by FIG. 2, the HLA-aware sequencing system 106 aligns the extracted nucleotide reads with HLA-allele-reference sequences 204. For instance, the HLA-aware sequencing system 106 aligns the extracted nucleotide reads with HLA-allele-reference sequences from the IPD-IMGT/HLA database. Accordingly, such an alignment process can include approximately 7,918 full-length HLA-allele-reference sequences. To accommodate such a significant number of reference sequences, in some cases, the HLA-aware sequencing system 106 groups the HLA-allele-reference sequences into batches of HLA-allele-reference sequences. The HLA-aware sequencing system 106 further aligns each extracted nucleotide read with each batched HLA-allele-reference sequence by following an order of an initial batch of HLA-allele-reference sequences through a final batch of HLA-allele-reference sequences. In some implementations, the HLA-aware sequencing system 106 likewise determines local alignment scores for candidate alignments between each extracted nucleotide read and each HLA-allele-reference sequence.

After aligning and scoring the extracted nucleotide reads, as further shown in FIG. 2, the HLA-aware sequencing system 106 selects a subset of nucleotide-read alignments based on alignment scores 206. To select such a subset of nucleotide-read alignments and a corresponding subset of nucleotide reads, in some embodiments, the HLA-aware sequencing system 106 applies various filtering criteria to the aligned and extracted nucleotide reads. For example, in some cases, the HLA-aware sequencing system 106 selects, from among the candidate alignments, alignments of nucleotide reads and individual HLA-allele-references sequences that exhibit highest local alignment scores from among local alignment scores. As a further example, in certain embodiments, the HLA-aware sequencing system 106 (i) determines, from among the candidate alignments, that particular nucleotide-read alignments between nucleotide reads and individual HLA-allele-references sequences exhibit local alignment scores and nucleobase lengths satisfying a score-length-combination threshold and (ii) selects the particular nucleotide-read alignments that satisfy the score-length-combination threshold.

Having selected a subset of nucleotide-read alignments, as further shown in FIG. 2, the HLA-aware sequencing system 106 groups individual nucleotide-read alignments into HLA equivalence classes 208. For instance, in some embodiments, the HLA-aware sequencing system 106 identifies nucleotide reads corresponding to the selected subset of nucleotide-read alignments that match or otherwise support different HLA alleles at two-field resolution. The HLA-aware sequencing system 106 further groups different HLA-allele-reference sequences into HLA equivalence classes when nucleotide reads equally match or support such different HLA-allele-reference sequences. Such an HLA equivalence class may include, for instance, a first HLA allele, a second HLA allele, and a third HLA allele at two-field resolution with which a group of nucleotide reads equally aligns or matches.

After grouping nucleotide-read alignments into HLA equivalence classes, as further shown in FIG. 2, the HLA-aware sequencing system 106 determines candidate HLA alleles using an EM algorithm 210. For instance, in some embodiments, the HLA-aware sequencing system 106 uses a two-stage EM algorithm at different resolutions. By performing a first stage of an EM algorithm on individual nucleotide reads grouped according to HLA equivalence classes, for instance, the HLA-aware sequencing system 106 determines a set of candidate allele probabilities that a genomic sample comprises a set of candidate HLA alleles at two-field resolution. By further performing a second stage of the EM algorithm on individual nucleotide reads re-grouped according to updated HLA equivalence classes, the HLA-aware sequencing system 106 determines another set of candidate allele probabilities that the genomic sample comprises another or same set of candidate HLA alleles at full resolution.

Based on the candidate HLA alleles, as further shown in FIG. 2, the HLA-aware sequencing system 106 generates genotype calls 212. In particular, the HLA-aware sequencing system 106 can generate genotype calls that the genomic sample comprises HLA alleles at different resolutions and at different loci based on the candidate HLA alleles. For example, in some embodiments, the HLA-aware sequencing system 106 determines the genomic sample comprises one or more HLA alleles at a locus for HLA-A. Indeed, by again performing some or all of the acts shown in FIG. 2 for multiple HLA loci, in some embodiments, the HLA-aware sequencing system 106 determines genotype calls that the genomic sample comprises (i) one or more HLA alleles at a locus for HLA-A, (ii) one or more HLA alleles at a locus for HLA-B, and (ii) one or more HLA alleles at a locus for HLA-C.

As indicated above, in some embodiments, the HLA-aware sequencing system 106 aligns each extracted nucleotide read corresponding to an HLA genomic region of a genomic sample with each HLA-allele-reference sequences. In accordance with one or more embodiments, FIG. 3 illustrates the HLA-aware sequencing system 106 grouping HLA-allele-reference sequences into batches, aligning extracted nucleotide reads (batch by batch) with the HLA-allele-reference sequences, and determining alignment scores for each nucleotide-read alignment.

As shown in FIG. 3, for instance, the HLA-aware sequencing system 106 identifies HLA-allele-reference sequences from an HLA database and groups the HLA-allele-reference sequences into batches 302a - 302n. This disclosure uses "n" as a suffix to certain reference numbers and ellipses in the figures to indicate multiple different versions or instances of an item (e.g., hundreds or thousands or more versions of an item) may be present between one numbered item (e.g., a batch 302b, a candidate alignment 402g) an end number of an item (e.g., a batch 302n, a candidate alignment 402n ). As an example of batching, in some embodiments, the HLA-aware sequencing system 106 groups approximately 7,918 full HLA-allele-reference sequences form the IPD-IMGT/HLA database into approximately 16 batches, where each batch includes approximately 500 HLA-allele-reference sequences. In some embodiments, however, the HLA-aware sequencing system 106 batches any number of HLA-allele-reference sequences into any number of batches.

As indicated by FIG. 3, in some embodiments, the HLA-aware sequencing system 106 integrates digital representations of the HLA-allele-reference sequences organized within the batches 302a - 302n into an HLA hash table 310. For instance, the HLA-aware sequencing system 106 associates, within the HLA hash table 310, nucleobase identifiers for nucleobases (e.g., single-letter codes, or letter codes representing multiple nucleobase types) from the HLA-allele-reference sequences with values representing genomic coordinates of a reference genome (e.g., genomic coordinates for GRCh38 corresponding to the HLA MHC complex). In some cases, the HLA-aware sequencing system 106 generates an HLA reference sequence file (e.g., FASTA format file) and/or an HLA reference alignment file (e.g., a Sequence Alignment/Map (SAM) file) comprising digitally encoded versions of the HLA-allele-reference sequences. The HLA-aware sequencing system 106 further uses the HLA reference sequence file and the HLA reference alignment file to associate, within the HLA hash table 310, nucleobase identifiers for nucleobases from the HLA-allele-reference sequences with values representing genomic coordinates of the reference genome. Although not depicted in FIG. 3, in some cases, the HLA hash table 310 differs from a separate hash table for a more general reference genome (e.g., covering all of GRCh38) used for variant calling.

As further shown in FIG. 3, in some embodiments, the HLA-aware sequencing system 106 generates nucleotide-read alignments 308 between extracted nucleotide reads 306 corresponding to an HLA genomic region and HLA-allele-reference sequences within the batches 302a - 302n. As indicated by FIG. 3, determining the nucleotide-read alignments 308 can comprise an iterative process by which the HLA-aware sequencing system 106 aligns each of the extracted nucleotide reads 306 with each HLA-allele-reference sequence in each of the batches 302a - 302n-batch by batch. When generating nucleotide-read alignments for a batch 302b, for instance, the HLA-aware sequencing system 106 aligns each of the extracted nucleotide reads 306 with each of HLA-allele-reference sequences 304a, 304b, and 304c, and up through an HLA-allele-reference sequence 304n from the batch 302b.

As indicated by FIG. 3, the HLA-aware sequencing system 106 can use the HLA hash table 310 to align the extracted nucleotide reads 306 with the HLA-allele-reference sequences within the batches 302a - 302n. For instance, the HLA-aware sequencing system 106 can use a hash function to transform a digital representation of an extracted nucleotide read to generate a hash. The HLA-aware sequencing system 106 further matches or maps the hash with a value corresponding to one or more HLA-allele-reference sequences within a batch. Batch by batch, the HLA-aware sequencing system 106 performs such a hash function for each extracted nucleotide read and determines values corresponding to HLA-allele-reference sequences.

As further shown in FIG. 3, either after or while generating the nucleotide-read alignments 308, the HLA-aware sequencing system 106 determines alignment scores for each of the nucleotide-read alignments 308. Such alignment scores can be determined after or while generating the nucleotide-read alignments 308 in batches. To illustrate, for a batch of the batches 302a - 302n, the HLA-aware sequencing system 106 determines an alignment score 312a for a nucleotide-read alignment 314a between an extracted nucleotide read and an HLA-allele-reference sequence. As indicated by FIG. 3, the HLA-aware sequencing system 106 continues to determine alignment scores for the batch until determining the alignment score 312n for a nucleotide-read alignment 314n between an extracted nucleotide read and an HLA-allele-reference sequence. As indicated above, in certain cases, the HLA-aware sequencing system 106 determines a Smith-Waterman score (or a version of a Smith-Waterman score) for an alignment score for such a nucleotide-read alignment.

After determining alignment scores for nucleotide read alignments, in some embodiments, the HLA-aware sequencing system 106 filters nucleotide-read alignments and groups different filtered subset of nucleotide-read alignments. In accordance with one or more embodiments, FIG. 4 illustrates the HLA-aware sequencing system 106 selecting a subset of nucleotide-read alignments based on various alignment-score thresholds and grouping the selected subset of nucleotide-read alignments into HLA equivalence classes. While FIG. 4 depicts filtering and grouping a subset of nucleotide-read alignments for a particular HLA locus (e.g., HLA-A), in some embodiments, the HLA-aware sequencing system 106 performs the same type of filtering and grouping of nucleotide-read alignments into HLA equivalence classes.

As shown in FIG. 4, identifies alignment scores for candidate alignments 402a, 402b, 402c, 402d, 402e, 402f, and 402g, and up through candidate alignment 402n between extracted nucleotide reads of a nucleotide read pair and HLA-allele-reference sequences and compares the candidate alignments 402a-402a to various thresholds. For example, the HLA-aware sequencing system 106 determines a subset of candidate alignments from the candidate alignments 402a-402n that satisfy a maximal alignment score threshold 404. From among the candidate alignments 402a-402n, in some cases, the HLA-aware sequencing system 106 identifies (and selects) candidate alignments between nucleotide read pairs and individual HLA-allele-references sequences that exhibit highest local alignment scores from among alignment scores.

To illustrate the maximal alignment score threshold 404, in some embodiments, the HLA-aware sequencing system 106 identifies (and selects) candidate alignments, from among HLA-allele-reference sequences that align with or "hit" nucleotide read pairs, that exhibit maximal local alignment scores. For example, the HLA-aware sequencing system 106 identifies (and selects) candidate alignments between nucleotide read pairs and HLA-allele-references sequences in which each mate of a nucleotide read pair exhibit a maximal local alignment score. As a further example, the HLA-aware sequencing system 106 identifies (and selects) candidate alignments between nucleotide read pairs and HLA-allele-references sequences in which an average local alignment score from both mates of a nucleotide read pair exhibit a maximal local alignment score.

In addition or in the alternative to the maximal alignment score threshold 404, in some embodiments, the HLA-aware sequencing system 106 determines a subset of candidate alignments from the candidate alignments 402a-402n that satisfy a score-length-combination threshold 406. In some cases, the score-length-combination threshold 406 constitutes a metric representing a combination of (i) a threshold alignment score for a nucleotide-read alignment between a nucleotide read and HLA-allele-reference sequence and (ii) a length of the nucleotide read in terms of nucleobases (e.g., 150 bases). For instance, the score-length-combination threshold 406 can be a product of (i) the threshold alignment score for a nucleotide-read alignment and (ii) the length of the nucleotide read. In particular, in some such cases, the score-length-combination threshold 406 can be represented as 0.67 * nucleotide read length (e.g., 140, 150, 160 bases), where 0.67 constitutes a threshold ratio used to filter alignments by their alignment score as a function of the nucleotide read length. Other alignment score threshold ratios may be used for filtering alignments, such as 0.50, 0.70, or 0.75 depending on assay type input data. If the nucleotide read is a mate from a nucleotide read pair, in some embodiments, both mates must satisfy the score-length-combination threshold 406. To satisfy the score-length-combination threshold 406, for instance, the nucleotide read must equal or exceed the score-length-combination threshold 406.

To illustrate the score-length-combination threshold 406, in some embodiments, the HLA-aware sequencing system 106 determines, from among the candidate alignments 402a-402n, that particular nucleotide-read alignments between nucleotide read pairs and individual HLA-allele-references sequences exhibit local alignment scores and nucleobase lengths satisfying the score-length-combination threshold 406. The HLA-aware sequencing system 106 further selects the particular nucleotide-read alignments that satisfy the score-length-combination threshold 406. By using the score-length-combination threshold 406, the HLA-aware sequencing system 106 can remove nucleotide-read alignments in which relatively shorter nucleotide reads exhibit alignment scores satisfying one or more alignment score thresholds but exhibit read lengths that reduce the informative value of the nucleotide reads in terms of alignment and HLA genotype calling.

In addition or in the alternative to the score-length-combination threshold 406, in some embodiments, the HLA-aware sequencing system 106 determines a subset of candidate alignments from the candidate alignments 402a-402n that satisfy a minimum alignment score threshold 408. From among the candidate alignments 402a-402n, for instance, the HLA-aware sequencing system 106 identifies (and selects) candidate alignments between nucleotide read pairs and individual HLA-allele-references sequences with local alignment scores that equal or exceed a minimum alignment score. In some cases, the minimum alignment score constitutes a Smith-Waterman score of 30 or 0.30. But the HLA-aware sequencing system 106 can use various other minimum alignment scores, such as 20, 25, or 35 in terms of Smith-Waterman or a version of Smith-Waterman.

As further shown in FIG. 4, in some embodiments, the HLA-aware sequencing system 106 selects a subset of nucleotide-read alignments that satisfy one or more of the maximal alignment score threshold 404, the score-length-combination threshold 406, or the minimum alignment score threshold 408. In some cases, for instance, the HLA-aware sequencing system 106 selects a subset of nucleotide-read alignments that satisfy each of the maximal alignment score threshold 404, the score-length-combination threshold 406, and the minimum alignment score threshold 408. From among the candidate alignments 402a-402n, for instance, the HLA-aware sequencing system 106 selects a subset of nucleotide-read alignments 409 comprising the candidate alignments 402a, 402b, 402c, and 402n. Because of space constraints and for illustrative purposes, FIG. 4 depicts the subset of nucleotide-read alignments 409 comprising four candidate alignments. In practice, however, the HLA-aware sequencing system 106 can select a subset of nucleotide-read alignments of various numbers.

After selecting the subset of nucleotide-read alignments 409, as further shown in FIG. 4, the HLA-aware sequencing system 106 groups nucleotide reads corresponding to the subset of nucleotide-read alignments 409 into HLA equivalence classes 410a-410n. As shown in FIG. 4, each of the HLA equivalence classes 410a-410n includes a group of one or more HLA alleles that are supported by a corresponding group of nucleotide reads. In particular, the HLA equivalence classes 410a, 410b, and 410n comprise HLA alleles 412a, 412b, and 412n, respectively. As further shown, groups of nucleotide reads 414a, 414b, and 414n equally support the HLA alleles 412a, 412b, and 412n, respectively. For instance, each nucleotide reads in a group of nucleotide reads 414a aligns with and satisfies the maximal alignment score threshold 404 for the HLA alleles 412a.

To further illustrate an HLA equivalence class, in some embodiments, a first subset of nucleotide read pairs exhibits maximal alignment scores when aligned with A*02:01:01:01, A*02:197:02, and A*02:197:01; a second subset of nucleotide read pairs exhibit maximal alignment scores when aligned with A*02:01:01:01 and A*02:197:01; a third subset of nucleotide read pairs exhibit maximal alignment scores when aligned with A*02:01:01:02L; and a fourth subset of nucleotide read pairs exhibit maximal alignment scores when aligned with A*02:07:01:01. The HLA-aware sequencing system 106 groups (i) A*02:01 and A*02:197 at two-field resolution into a first HLA equivalence class, as supported by the first and second subsets of nucleotide read pairs; (ii) A*02:01 at two-field resolution into a second HLA equivalence class, as supported by the third subset of nucleotide read pairs; and (iii) A*02:07 at two-field resolution into a third HLA equivalence class, as supported by the fourth subset of nucleotide read pairs. While this disclosure frequently uses candidate HLA alleles A*02:01:01:01, A*02:197:02, and A*02:197:01, the HLA-aware sequencing system 106 can determine candidate allele probabilities and genotype calls for various other HLA alleles at varying resolutions (e.g., B*15:02, C*01:02:01:01, C*03:04, DRB1*13:02).

Based on filtered nucleotide reads grouped according to HLA equivalence classes, as suggested above, the HLA-aware sequencing system 106 can perform an EM algorithm to determine candidate allele probabilities. In accordance with one or more embodiments, FIGS. 5A-5C illustrate the HLA-aware sequencing system 106 performing an EM algorithm to determine posterior candidate allele probabilities that a genomic sample comprises candidate HLA alleles based on nucleotide reads grouped in HLA equivalence classes. As indicated in FIGS. 5A-5C, the HLA-aware sequencing system 106 redistributes nucleotide reads based on different scenarios of the nucleotide reads supporting various HLA alleles. For purposes of illustration and space constraints, FIGS. 5A-5C depict only a few candidate HLA alleles and example numbers of supporting nucleotide reads. In practice, however, the HLA-aware sequencing system 106 can determine candidate allele probabilities for various numbers of candidate HLA alleles.

As shown in FIG. 5A, for example, the HLA-aware sequencing system 106 organizes observed read data into an observed data table 502 based on HLA equivalence classes. In particular, the HLA-aware sequencing system 106 identifies candidate HLA alleles from the HLA equivalence classes and determines a number of nucleotide reads supporting the candidate HLA alleles (e.g., exhibiting a maximal alignment score when aligned with a particular candidate HLA allele). As shown in the observed data table 502, for instance, the HLA-aware sequencing system 106 determines that 100 nucleotide reads support A*02:01:01:01, 101 nucleotide reads support A*02:01:01:06, and 102 nucleotide reads support A*02:07:01:01. The numbers of supporting nucleotide reads in the observed data table 502 represent an initial distribution of nucleotide reads among candidate HLA alleles: A*02:01:01:01, A*02:01:01:06, and A*02:07:01:01. Critically, some nucleotide reads could support more than one candidate HLA alleles among A*02:01:01:01, A*02:01:01:06, and A*02:07:01:01.

Based on the initial distribution of nucleotide reads from the observed read data, as further shown in the observed data table 502, the HLA-aware sequencing system 106 determines initial candidate allele probabilities. From the observed read data, for instance, the HLA-aware sequencing system 106 determines an initial candidate allele probability of approximately 33% that a genomic sample comprises A*02:01:01:01, an initial candidate allele probability of approximately 33% that the genomic sample comprises A*02:01:01:06, and an initial candidate allele probability of approximately 34% that the genomic sample comprises A*02:07:01:01.

From the initial distribution of nucleotide reads, as further shown in FIG. 5A, the HLA-aware sequencing system 106 distributes the nucleotide reads into a first scenario 504 for a first iteration of the EM algorithm. The first scenario 504 represents a possible distribution of nucleotide reads among candidate HLA alleles that would explain the observed read data. In particular, the HLA-aware sequencing system 106 distributes the nucleotide reads into a first read group 506a of nucleotide reads supporting A*02:01:01:01, a second read group 506b of nucleotide reads supporting A*02:01:01:06, and a third read group 506c of nucleotide reads supporting A*02:07:01:01.

Under the first scenario 504, as shown in FIG. 5A, the HLA-aware sequencing system 106 determines that 143 nucleotide reads could be distributed among the first read group 506a, the second read group 506b, and the third read group 506c to explain or represent the observed read data. As part of that distribution, 60 nucleotide reads from the total 143 support each of the three candidate HLA alleles. Indeed, as shown in FIG. 5A, the HLA-aware sequencing system 106 further determines that 60 of the 100 nucleotide reads supporting A*02:01:01:01 in the observed read data could also support A*02:01:01:06 and A*02:07:01:01. By contrast, the HLA-aware sequencing system 106 determines that 40, 1, and 2 nucleotide reads could uniquely support A*02:01:01:01, A*02:01:01:06, and A*02:07:01:01, respectively, under the first scenario 504.

Based on the first scenario 504, the HLA-aware sequencing system 106 redistributes the nucleotide reads to hypothesize which candidate HLA allele from A*02:01:01:01, A*02:01:01:06, and A*02:07:01:01 could be best supported or aligned with the 60 multi-allele-supporting nucleotide reads to potentially explain the observed read data-assuming an equal probability among the candidate HLA alleles. As shown in an updated data table 508, for example, the HLA-aware sequencing system 106 redistributes 20 nucleotide reads from the 60 multi-allele-supporting nucleotide reads to each of A*02:01:01:01, A*02:01:01:06, and A*02:07:01:01. Such a redistribution separates multi-allele-supporting nucleotide reads that presumably have an equal probability of best supporting the three candidate HLA alleles. After redistributing each of the 60 nucleotide reads to a single candidate HLA alleles, as shown in the updated data table 508, 60 nucleotide reads support A*02:01:01:01, 41 nucleotide reads support A*02:01:01:06, and 42 nucleotide reads support A*02:07:01:01.

Based on the redistribution of nucleotide reads shown in the updated data table 508, as further shown in FIG. 5A, the HLA-aware sequencing system 106 determines updated candidate allele probabilities according to the first scenario 504. In particular, the HLA-aware sequencing system 106 determines an updated candidate allele probability of approximately 42% that the genomic sample comprises A*02:01:01:01, an updated candidate allele probability of approximately 29% that the genomic sample comprises A*02:01:01:06, and an updated candidate allele probability of approximately 29% that the genomic sample comprises A*02:07:01:01. The updated candidate allele probabilities in the updated data table 508 merely represent one iteration of candidate allele probabilities that could explain the observed read data.

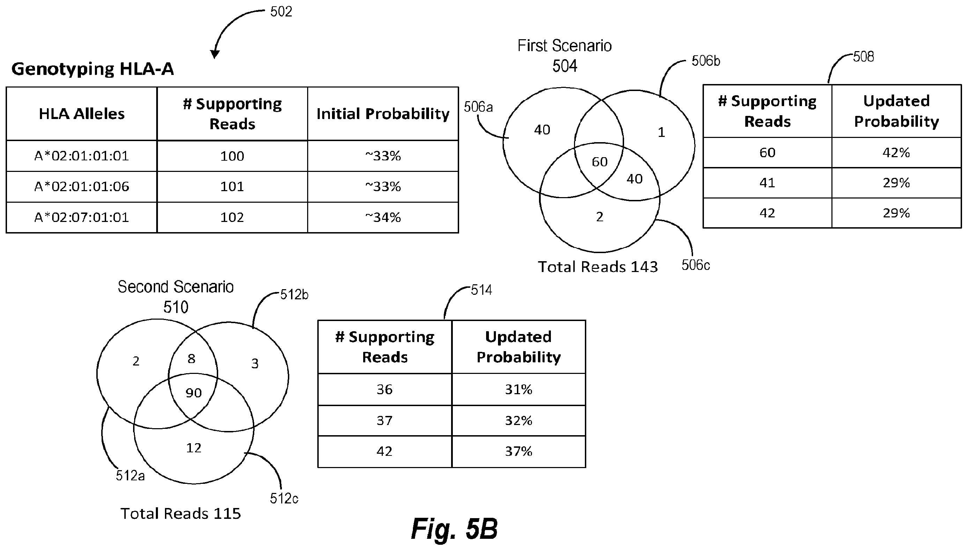

In addition to the first iteration of the EM algorithm, as shown in FIG. 5B, the HLA-aware sequencing system 106 performs a second iteration of the EM algorithm. Again from the initial distribution of nucleotide reads, the HLA-aware sequencing system 106 distributes the nucleotide reads into a second scenario 510 for the second iteration of the EM algorithm. Similar to the first scenario 504, the second scenario 510 represents a possible distribution of nucleotide reads among candidate HLA alleles that would explain the observed read data. As indicated in FIG. 5B, the HLA-aware sequencing system 106 distributes the nucleotide reads into a first read group 512a of nucleotide reads supporting A*02:01:01:01, a second read group 512b of nucleotide reads supporting A*02:01:01:06, and a third read group 512c of nucleotide reads supporting A*02:07:01:01.

In contrast to the first scenario 504, the HLA-aware sequencing system 106 determines that 115 nucleotide reads could be distributed among the first read group 512a, the second read group 512b, and the third read group 512c to explain or represent the observed read data. As part of that distribution, 90 nucleotide reads from the total 115 support each of the three candidate HLA alleles. Indeed, as shown in FIG. 5B, the HLA-aware sequencing system 106 further determines that 90 of the 100 nucleotide reads supporting A*02:01:01:01 in the observed read data could also support A*02:01:01:06 and A*02:07:01:01. By contrast, the HLA-aware sequencing system 106 determines that 2, 3, and 12 nucleotide reads could uniquely support A*02:01:01:01, A*02:01:01:06, and A*02:07:01:01, respectively, under the second scenario 510.

Based on the second scenario 510, the HLA-aware sequencing system 106 redistributes the nucleotide reads to hypothesize which candidate HLA allele from A*02:01:01:01, A*02:01:01:06, and A*02:07:01:01 could be best supported or aligned with the 90 multi-allele-supporting nucleotide reads to potentially explain the observed read data-again assuming an equal probability among the candidate HLA alleles. As shown in an updated data table 514, for example, the HLA-aware sequencing system 106 redistributes 30 nucleotide reads from the 90 multi-allele-supporting nucleotide reads to each of A*02:01:01:01, A*02:01:01:06, and A*02:07:01:01. After redistributing each of the 90 nucleotide reads to a single candidate HLA alleles, as shown in the updated data table 514, 36 nucleotide reads support A*02:01:01:01, 37 nucleotide reads support A*02:01:01:06, and 42 nucleotide reads support A*02:07:01:01.

Based on the redistribution of nucleotide reads shown in the updated data table 514, the HLA-aware sequencing system 106 determines updated candidate allele probabilities according to the second scenario 510. In particular, the HLA-aware sequencing system 106 determines an updated candidate allele probability of approximately 31% that the genomic sample comprises A*02:01:01:01, an updated candidate allele probability of approximately 32% that the genomic sample comprises A*02:01:01:06, and an updated candidate allele probability of approximately 37% that the genomic sample comprises A*02:07:01:01.

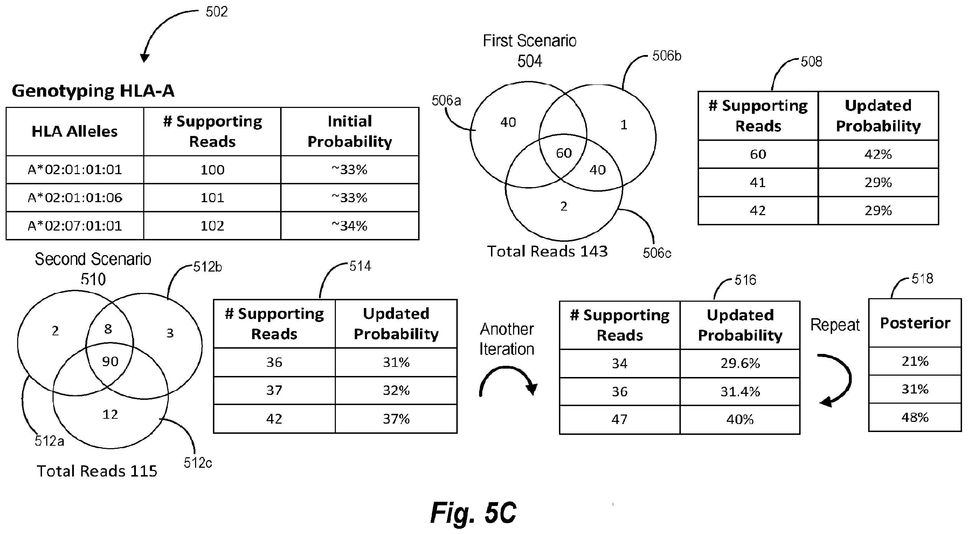

As indicated by FIG. 5C, the HLA-aware sequencing system 106 continues to perform iterations of the EM algorithm with new scenarios. In each such iteration, the HLA-aware sequencing system 106 (i) distributes nucleotide reads from the observed read data into new scenarios with groups of supporting nucleotide reads for the candidate HLA alleles, (ii) redistributes the nucleotide reads under the new scenarios to individual candidate HLA alleles, and (iii) determines updated candidate allele probabilities according to the new scenarios. As indicated by FIG. 5C, across iterations, the EM algorithm effectively redistributes nucleotide reads to place more weight on a candidate HLA allele with more nucleotide reads that uniquely support the candidate HLA allele than other candidate HLA alleles.

As shown in an updated data table 516 in FIG. 5C, for example, the HLA-aware sequencing system 106 redistributes nucleotide reads under a new scenario to support individual candidate HLA alleles. After redistributing nucleotide reads, as shown in the updated data table 516, 34 nucleotide reads support A*02:01:01:01, 36 nucleotide reads support A*02:01:01:06, and 47 nucleotide reads support A*02:07:01:01. Based on the redistribution of nucleotide reads, as shown in the updated data table 516, the HLA-aware sequencing system 106 likewise determines an updated candidate allele probability of approximately 29.6% that the genomic sample comprises A*02:01:01:01, an updated candidate allele probability of approximately 31.4% that the genomic sample comprises A*02:01:01:06, and an updated candidate allele probability of approximately 40% that the genomic sample comprises A*02:07:01:01.

As further indicated by FIG. 5C, the HLA-aware sequencing system 106 performs iterations of the EM algorithm until satisfying a convergence criteria. In some embodiments, for instance, the HLA-aware sequencing system 106 performs iterations until updated candidate allele probabilities among the candidate HLA alleles do not change more than a threshold probability difference (e.g., 10⁻⁶, 10⁻⁴) across iterations (e.g., as determined by L2 norm). For instance, if the change in candidate allele probabilities between updated candidate allele probabilities of neighboring iterations is less than 10⁻⁶, the candidate allele probabilities have converged. Additionally or alternatively, the HLA-aware sequencing system 106 performs iterations until reaching a predetermined number of iterations for a particular stage (e.g., 100 iterations, 200 iterations).

After satisfying the convergence criteria, as further shown in FIG. 5C, the HLA-aware sequencing system 106 generates posterior candidate allele probabilities 518. Such posterior candidate allele probabilities represent the candidate allele probabilities to which the EM algorithm converged. In some cases, the posterior candidate allele probabilities are the updated candidate allele probabilities of a last EM algorithm iteration for a particular stage. In this particular example, the HLA-aware sequencing system 106 likewise determines a posterior candidate allele probability of approximately 21% that the genomic sample comprises A*02:01:01:01, a posterior candidate allele probability of approximately 31% that the genomic sample comprises A*02:01:01:06, and a posterior candidate allele probability of approximately 48% that the genomic sample comprises A*02:07:01:01. As indicated by the change from the initial candidate allele probabilities and the posterior candidate allele probabilities, by performing the EM algorithm, the HLA-aware sequencing system 106 effectively redistributes nucleotide reads to place more weight on candidate HLA allele A*02:07:01:01 because more nucleotide reads uniquely support A*02:07:01:01 than A*02:01:01:01 and A*02:01:01:06.

In some embodiments, the HLA-aware sequencing system 106 performs a likelihood function as part of an EM algorithm. As background for such a likelihood function, for a given genomic sample, the HLA-aware sequencing system 106 receives observed read data **X**. Based on the observed read data **X**, the HLA-aware sequencing system 106 organizes a nucleotide-read set into an HLA hit matrix **C**, where C*ᵢⱼ* includes a value of 1 or a value of 0 indicating whether nucleotide read *i* aligns or does not align with HLA allele *j*, respectively. In performing an EM algorithm, the HLA-aware sequencing system 106 uses the likelihood function to maximize-or determine a maximum likelihood estimation (MLE) for-unknown parameters 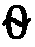 , where 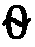 represents a probability of drawing a nucleotide read from an HLA allele *j*. In other words, for each HLA allele *j*, the HLA-aware sequencing system 106 determines an abundance 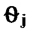 of HLA allele *j*. While the HLA-aware sequencing system 106 does not receive data identifying an HLA of origin for each nucleotide read, the HLA-aware sequencing system 106 can nevertheless perform a likelihood function based on a number of nucleotide reads *nⱼ* attributed to HLA allele *j*.

Based on the parameters outlined above, in some embodiments, the HLA-aware sequencing system 106 uses the following likelihood function to determine MLE for 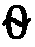 : $L \left(θ; C\right) = p \left(C\left|θ\right.\right) = {\prod }_{i = 1}^{Reads} {\sum }_{j = 1}^{Alleles} θj ∗ Cij$

To determine MLE for using function (1), in some embodiments, the HLA-aware sequencing system 106 performs both an expectation (E) step and a maximization (M) step as part of the EM algorithm. In particular, the HLA-aware sequencing system 106 performs the E step and the M step according to functions (2) and (3) below, respectively: $for each allel , nj = {\sum }_{i = 1}^{Reads} \frac{θ j}{{\sum }_{k = 1}^{Alleles} Ci k θ k} Cij$ ${θ}_{j} = \frac{nj}{{\sum }_{k = 1}^{Alleles} nk}$

In addition to performing the E step and M step set forth in functions (2) and (3) in an iteration of the EM algorithm, in some embodiments, the HLA-aware sequencing system 106 further removes (or culls) certain candidate HLA alleles that lack a threshold proportion of supporting nucleotide reads during EM algorithm iterations. For example, after a threshold number of iterations-such as 10, 15, or 20 iterations-the HLA-aware sequencing system 106 removes candidate HLA alleles from consideration that do not exhibit at least a candidate allele probability of 10%. Other threshold proportions may be used, such as 5%, 15%, or 20%. By removing candidate HLA alleles that fail to satisfy such a threshold proportion of supporting nucleotide reads, in some embodiments, the HLA-aware sequencing system 106 narrows a set of candidate HLA alleles across iterations.

As indicated above, in some embodiments, the HLA-aware sequencing system 106 performs two different stages of an EM algorithm at different HLA allele resolutions to determine candidate allele probabilities. In accordance with one or more embodiments, FIG. 6 illustrates the HLA-aware sequencing system 106 (i) performing a first EM algorithm stage to determine a first set of candidate allele probabilities that a genomic sample includes candidate HLA alleles at two-field resolution (ii) performing a second EM algorithm stage to determine a second set of candidate allele probabilities that the genomic sample includes candidate HLA alleles at full resolution. The HLA-aware sequencing system 106 can subsequently determine genotype calls for HLA alleles based on posterior candidate allele probabilities from the second EM algorithm stage.

As shown in FIG. 6, for instance, the HLA-aware sequencing system 106 identifies or determines HLA equivalence classes 602a-602n. For example, the HLA-aware sequencing system 106 identifies different sets of filtered nucleotide reads aligning with or otherwise supporting different groups of HLA alleles, where some such filtered nucleotide reads align with or support multiple HLA alleles. Based on the different sets of filtered nucleotide reads, the HLA-aware sequencing system 106 groups one or more HLA alleles into the HLA equivalences classes 602a-602n according to the subsets of filtered nucleotide reads supporting each group of HLA alleles. As illustrated by FIG. 6, each of HLA equivalence classes 602a, 602b, and 602n include HLA allele(s) and a subset of filtered nucleotide reads that align with the corresponding HLA allele(s). After grouping, the HLA-aware sequencing system 106 inputs the filtered nucleotide reads grouped according to the HLA equivalence classes 602a-602n at two-field resolution into an expectation-maximation (EM) algorithm.

To repurpose an example from above for the HLA equivalence classes 602a-602n, in some embodiments, the HLA-aware sequencing system 106 identifies a first subset of nucleotide read pairs exhibits maximal alignment scores when aligned with A*02:01:01:01, A*02:197:02, and A*02:197:01; a second subset of nucleotide read pairs exhibit maximal alignment scores when aligned with A*02:01:01:01 and A*02:197:01; a third subset of nucleotide read pairs exhibit maximal alignment scores when aligned with A*02:01:01:02L; and a fourth subset of nucleotide read pairs exhibit maximal alignment scores when aligned with A*02:07:01:01.

The HLA-aware sequencing system 106 subsequently groups (i) A*02:01 and A*02:197 at two-field resolution into a first HLA equivalence class, as supported by the first and second subsets of nucleotide read pairs; (ii) A*02:01 at two-field resolution into a second HLA equivalence class, as supported by the third subset of nucleotide read pairs; and (iii) A*02:07 at two-field resolution into a third HLA equivalence class, as supported by the fourth subset of nucleotide read pairs. The first, second, and third HLA equivalence classes represent examples of the HLA equivalence classes 602a-602n.

Based on the filtered nucleotide reads grouped according to the HLA equivalence classes 602a-602n at two-field resolution, as indicated by FIG. 6, the HLA-aware sequencing system 106 performs an EM algorithm at two-field resolution 604. In some embodiments, the HLA-aware sequencing system 106 finishes the EM algorithm at two-field resolution 604 after satisfying a convergence criteria (e.g., no change more than 10⁻⁶ between iterations as determined by L2 norm). Consistent with the disclosure above, the HLA-aware sequencing system 106 determines a first set of candidate allele probabilities for the HLA alleles grouped into the HLA equivalence classes 602a-602n. As indicated above, the first set of candidate allele probabilities may take the form of posterior candidate allele probabilities output by the EM algorithm at two-field resolution 604.

As shown in an allele probability table 606, for instance, the HLA-aware sequencing system 106 determines a posterior candidate allele probability of approximately 70% that a genomic sample comprises A*02:01, a posterior candidate allele probability of approximately 19% that the genomic sample comprises A*02:07, and a posterior candidate allele probability of approximately 5% that the genomic sample comprises A*02:197. Although not shown due to space constraints, the HLA-aware sequencing system 106 may determine additional posterior candidate allele probabilities at two-field resolution. In some embodiments, the HLA-aware sequencing system 106 removes HLA alleles that fail to satisfy a threshold posterior candidate allele probability for a first EM algorithm stage. Such a threshold posterior candidate allele probability may constitute 15%, 18%, 20%, etc. Based on the candidate HLA allele of A*02:197 failing to satisfy such a threshold posterior candidate allele probability at two-field resolution, for instance, the HLA-aware sequencing system 106 removes A*02:197 from consideration for the second stage of EM algorithm.

After determining the first set of candidate allele probabilities shown in the allele probability table 606, as further shown in FIG. 6, the HLA-aware sequencing system 106 extends the candidate HLA alleles from two-field resolution to full resolution and regroups remaining candidate HLA alleles into HLA equivalence classes 608a-608n, as supported by filtered subsets of nucleotide reads. In contrast to the HLA equivalence classes 602a-602n, the HLA equivalence classes 608a-608n group candidate HLA alleles at full resolution. As illustrated by FIG. 6, each of HLA equivalence classes 608a, 608b, and 608n include HLA allele(s) and a subset of filtered nucleotide reads that align with the corresponding HLA allele(s). After regrouping the subset of filtered nucleotide reads, the HLA-aware sequencing system 106 inputs the filtered nucleotide reads grouped according to the HLA equivalence classes 608a-608n at full-field resolution into the second EM algorithm stage.

As an example of such regrouping, in some embodiments, the HLA-aware sequencing system 106 regroups HLA alleles at full resolution based on the posterior candidate allele probabilities for A*02:01, A*02:07, and A*02:197 described above. After removing A*02:197 for failing a threshold posterior candidate allele probability, the HLA-aware sequencing system 106 regroups into the following HLA equivalence classes: (i) A*02:01:01:01 at full resolution into a first HLA equivalence class, as supported by the first and second subsets of nucleotide read pairs; (ii) A*02:01:01:02L at full resolution into a second HLA equivalence class, as supported by the third subset of nucleotide read pairs; and (iii) A*02:07:01:01 at full resolution into a third HLA equivalence class, as supported by the fourth subset of nucleotide read pairs. The first, second, and third HLA equivalence classes represent examples of the HLA equivalence classes 608a-608n.

Based on the filtered nucleotide reads regrouped according to the HLA equivalence classes 608a-608n at full resolution, as indicated by FIG. 6, the HLA-aware sequencing system 106 performs an EM algorithm at full resolution 610. Consistent with the disclosure above, the HLA-aware sequencing system 106 determines a second set of candidate allele probabilities for the HLA alleles grouped into the HLA equivalence classes 608a-608n. As indicated above, the second set of candidate allele probabilities may take the form of posterior candidate allele probabilities output by the EM algorithm at full resolution 610.

As shown in an allele probability table 612, for instance, the HLA-aware sequencing system 106 determines a posterior candidate allele probability of approximately 52% that a genomic sample comprises A*02:01:01:01, a posterior candidate allele probability of approximately 18% that the genomic sample comprises A*02:0:01:01:02L, and a posterior candidate allele probability of approximately 3% that the genomic sample comprises A*02:07:01:01. Although not shown due to space constraints, the HLA-aware sequencing system 106 may determine additional posterior candidate allele probabilities at full resolution. As explained further below, in some embodiments, the HLA-aware sequencing system 106 removes HLA alleles that fail to satisfy a threshold posterior candidate allele probability for a second EM algorithm stage. By removing or keeping candidate HLA alleles based on a threshold posterior candidate allele probability for the second EM algorithm stage, in some embodiments, the HLA-aware sequencing system 106 determines candidate HLA alleles to remove or keep for heterozygous or homozygous genotype calls.

As indicated above, the HLA-aware sequencing system 106 can generate genotype calls for HLA alleles at one or both of two-field resolution and full resolution. In accordance with one or more embodiments, FIG. 7 illustrates the HLA-aware sequencing system 106 determining whether to generate HLA genotype calls for a genomic sample at either two-field resolution or full resolution. For purposes of illustration and space constraints, FIG. 7 depicts genotype calls for HLA-A alleles at an HLA-A locus. In some embodiments, however, the HLA-aware sequencing system 106 generates genotype calls for various HLA alleles at one or more various HLA loci (e.g., genotype calls for a genomic sample at each of HLA-A, HLA-B, and HLA-C loci).

As an overview of FIG. 7, the HLA-aware sequencing system 106 selects a proposed HLA allele(s) 706 based on posterior candidate allele probabilities at two-field resolution determined by a first EM algorithm stage. In examples below, this disclosure describes circumstances in which the proposed HLA allele(s) 706 comprises a top two candidate HLA alleles. In some embodiments, however, the proposed HLA allele(s) 706 comprise more or fewer candidate HLA alleles. If the HLA-aware sequencing system 106 determines the proposed HLA allele(s) 706 is among a top two candidate HLA alleles at full resolution that exhibit the highest candidate allele probabilities (e.g., top two posterior candidate allele probabilities), the HLA-aware sequencing system 106 generates a genotype call for the proposed HLA allele(s) 706 at full resolution. If, by contrast, the HLA-aware sequencing system 106 determines the proposed HLA allele(s) 706 is not among the top two candidate HLA alleles at full resolution, the HLA-aware sequencing system 106 generates a genotype call for the proposed HLA allele(s) 706 at two-field resolution.

As shown in FIG. 7, the HLA-aware sequencing system 106 determines candidate allele probabilities for candidate HLA alleles at a particular locus by performing two different stages of an EM algorithm at different HLA allele resolutions. By performing a first EM algorithm stage, consistent with the disclosure above, the HLA-aware sequencing system 106 determines posterior candidate allele probabilities of a genomic sample comprising candidate HLA alleles at two-field resolution shown in an allele probability table 702. By performing a second EM algorithm stage, the HLA-aware sequencing system 106 determines posterior candidate allele probabilities of a genomic sample comprising candidate HLA alleles at full resolution shown in an allele probability table 704.

As further shown in FIG. 7, the HLA-aware sequencing system 106 selects the proposed HLA allele(s) 706 based on the posterior candidate allele probabilities at two-field resolution shown in an allele probability table 702. For example, in some embodiments, the HLA-aware sequencing system 106 determines a short list of candidate HLA alleles based on the posterior candidate allele probabilities at two-field resolution. In certain implementations, the short list of candidate HLA alleles includes a top two candidate HLA alleles exhibiting the highest two posterior candidate allele probabilities. By contrast, in some cases, the short list of candidate HLA alleles includes three or more top candidate HLA alleles exhibiting the highest three or more posterior candidate allele probabilities. Regardless of the count for the proposed HLA allele(s) 706, as shown in FIG. 7, the HLA-aware sequencing system 106 determines whether to generate genotype calls at two-field resolution or full resolution from the proposed HLA allele(s) 706-or from alternative HLA alleles-based on decision nodes 708, 714, and 715 and optionally based on decision node 716. The following paragraphs describe the decision nodes 708, 714, 715, and 716.

As suggested above, in some embodiments, the HLA-aware sequencing system 106 generates genotype calls for HLA alleles exclusively at two-field resolution. In such embodiments, the HLA-aware sequencing system 106 uses the decision node 708 without the decision nodes 714, 715, and 716. As shown at the decision node 708, for instance, the HLA-aware sequencing system 106 determines whether the top two candidate HLA alleles from the proposed HLA allele(s) 706 satisfy a probability threshold. In some cases, the probability threshold constitutes a 20% posterior candidate allele probability that a genotype call exhibits the proposed HLA allele(s) 706. But the probability threshold may also constitute other posterior candidate allele probabilities, such as 15%, 25%, 30%, or some other percentage for posterior candidate allele probability.

As further shown in FIG. 7, if the top two candidate HLA alleles from the proposed HLA allele(s) 706 satisfy the probability threshold, the HLA-aware sequencing system 106 generates a heterozygous genotype call at two-field resolution 710. For example, the HLA-aware sequencing system 106 generates a genotype call that a genomic sample comprises A*02:01 and A*02:07 at an HLA-A locus. If, however, one candidate HLA allele of the top two candidate HLA alleles from the proposed HLA allele(s) 706 do not satisfy the probability threshold, the HLA-aware sequencing system 106 generates a homozygous genotype call at two-field resolution 712. For example, the HLA-aware sequencing system 106 generates a genotype call that the genomic sample comprises A*02:01 at the HLA-A locus. Based on the posterior candidate allele probabilities reported in the allele probability table 702 and a probability threshold of 20%, for example, the HLA-aware sequencing system 106 would generate a homozygous genotype call of A*02:01.

As further suggested above, in some embodiments, the HLA-aware sequencing system 106 generates genotype calls for HLA alleles either at two-field resolution or full-field resolution. In such embodiments, the HLA-aware sequencing system 106 uses the decision nodes 708, 714, 715, and 716, beginning with the decision node 714. As shown at the decision node 714, for instance, the HLA-aware sequencing system 106 determines whether the proposed HLA allele(s) 706 are among a top two candidate HLA alleles at full resolution. In particular, the HLA-aware sequencing system 106 determines whether the proposed HLA allele(s) 706 at two-field resolution include either of the top two candidate HLA alleles at full resolution based on the posterior candidate allele probabilities determined by the second EM algorithm stage. As noted above, the allele probability table 704 reports the posterior candidate allele probabilities determined by the second EM algorithm stage.

As further shown in FIG. 7, the HLA-aware sequencing system 106 determines at the decision node 714 whether the proposed HLA allele(s) 706 are among the top two candidate HLA alleles at full resolution. If the proposed HLA allele(s) 706 are among the top two candidate HLA alleles at full resolution-that is, "Yes" at decision node 714-the HLA-aware sequencing system 106 generates a genotype call at full resolution and optionally moves to the decision node 716.

If, however, one of the proposed HLA allele(s) 706 is not among the top two candidate HLA alleles at full resolution -that is, "No" at decision node 714-the HLA-aware sequencing system 106 determines at decision node 715 whether a candidate HLA allele at full resolution exhibits a higher candidate allele probability than the proposed HLA allele(s) 706 lacking support. As indicated by FIG. 7, if a candidate HLA allele at full resolution exhibits a higher posterior candidate allele probability than the proposed HLA allele(s) 706 lacking support-that is, "Yes" at decision node 715-the HLA-aware sequencing system 106 uses the candidate HLA allele at full resolution for genotype calling at full resolution. In such an example, the HLA-aware sequencing system 106 generates a genotype call using an HLA allele at full resolution, where one of the top two candidate HLA alleles at full resolution replaces the least supported HLA allele of the proposed HLA allele(s) 706 (here, two proposed HLA alleles).

If, by contrast, the candidate HLA allele at full resolution does not exhibit a higher posterior candidate allele probability than the proposed HLA allele(s) 706 lacking support-that is, "No" at decision node 715-the HLA-aware sequencing system 106 uses the proposed HLA allele(s) 706 at two-field resolution for genotype calling. When there are no candidates at full resolution to replace the least supported HLA allele of the proposed HLA allele(s) 706 at two-field resolution, then the HLA-aware sequencing system 106 generates a genotype call at two-field resolution and considers whether the proposed HLA allele(s) 706 satisfy the probability threshold at the decision node 708. Under the latter circumstance, the HLA-aware sequencing system 106 determines either the heterozygous genotype call at two-field resolution 710 or the homozygous genotype call at two-field resolution 712 as described above based on the decision node 708.

As further shown in FIG. 7, in some embodiments, the HLA-aware sequencing system 106 uses the decision node 716 to determine between reporting a heterozygous genotype call and a homozygous genotype call at full resolution-when one or more of the proposed HLA allele(s) 706 are among the top two candidate HLA alleles at full resolution. As shown at the decision node 716, the HLA-aware sequencing system 106 determines whether the top two candidate HLA alleles at full resolution satisfy a probability threshold. As above for the decision node 708, the probability threshold for the decision node 716 can constitute a 15%, 20%, 25%, 30%, or some other percentage for posterior candidate allele probability that a genotype call exhibits the proposed HLA allele(s) 706.

As further indicated by FIG. 7, if the top two candidate HLA alleles at full resolution satisfy the probability threshold at the decision node 716, the HLA-aware sequencing system 106 generates a heterozygous genotype call at full resolution 718. For example, the HLA-aware sequencing system 106 generates a genotype call that a genomic sample comprises A*02:01:01:01 and A*02:01:01:02L at an HLA-A locus. If, however, the top two candidate HLA alleles at full resolution do not satisfy the probability threshold, the HLA-aware sequencing system 106 generates a homozygous genotype call at full resolution 720. For example, the HLA-aware sequencing system 106 generates a genotype call that the genomic sample comprises A*02:01:01:01 at the HLA-A locus. Based on the posterior candidate allele probabilities reported in the allele probability table 704 and a probability threshold of 20%, for example, the HLA-aware sequencing system 106 would generate a homozygous genotype call of A*02:01:01:01.

Regardless of whether the HLA-aware sequencing system 106 generates a genotype call for HLA allele(s) at two-field resolution or full resolution, in some embodiments, the HLA-aware sequencing system 106 generates one or both of an HLA report file 722 and an HLA metrics file 724. For example, in some embodiments, the HLA report file 722 reports the HLA genotype calls for a genomic sample at either two-field or full-resolution. In some cases, the HLA report file 722 includes genotype calls of HLA alleles at multiple HLA loci (e.g., HLA-A locus, HLA-B locus, HLA-C locus; HLA-DR locus, HLA-DQ locus, HLA-DP locus). As a further example, the HLA metrics file 724 includes metrics for one or more candidate HLA alleles. Such metrics may include posterior candidate allele probabilities at either two-field resolution or full-field resolution and/or identify nucleotide reads that support each reported HLA allele from a genotype call at either two-field resolution or full resolution.

Independent of whether genotype calls are at two-field resolution or full resolution, as noted above, the HLA-aware sequencing system 106 can improve an accuracy of HLA genotyping as part of different types of sequencing assays. In accordance with one or more embodiments, FIG. 8 illustrates tables showing an accuracy, sensitivity, or specificity with which the HLA-aware sequencing system 106 generates HLA genotype calls for whole genome sequencing (WGS), whole exome sequencing (WES), and tumor gene panels. As indicated by tables 800a, 800b, and 800c, the HLA-aware sequencing system 106 demonstrates improved HLA genotyping accuracy over existing sequencing systems in each of WGS, WES, and tumor gene panels.

To test the accuracy of an embodiment of the HLA-aware sequencing system 106, researchers used the HLA-aware sequencing system 106 to generate HLA genotype calls for WGS, WES, and TruSight^{®} Oncology (TSO500). The researchers further compared the HLA genotype calls to HISAT2 genotypes as ground truths for WGS, histogenetics as ground truths for WES, and HISAT2 genotypes or IHWG sequences as ground truths for TSO500. As set forth below, researchers determined accuracy as (i) a count of all HLA genotype calls matching their corresponding ground truth HLA alleles (ii) divided by the total number of HLA genotype calls (namely, six genotype calls per individual genomic sample, with two genotype calls per HLA locus, and three loci evaluated: HLA-A, HLA-B and HLA-C).

As shown in the table 800a, the HLA-aware sequencing system 106 generated HLA genotyping calls with approximately 99.7% accuracy for overall WGS, a full sample-level accuracy of approximately 98.3% for WGS, and a homozygosity HLA genotype call accuracy of approximately 99.1%. In comparison to the accuracy of HLA genotyping calls from existing sequencing systems, as described above, the HLA-aware sequencing system 106 improves overall WGS by approximately 6.6% accuracy, improves full sample-level by approximately 31.1% accuracy, and improves homozygosity genotype calls by approximately 3.7% accuracy.

As shown in the table 800b, the HLA-aware sequencing system 106 generated HLA genotyping calls with approximately 95.7% sensitivity and 99.7% specificity for homozygosity HLA genotype calls. Such homozygosity HLA genotype calls exhibit an approximately 6.5% improvement in sensitivity and approximately 3.6% improvement in specificity over existing sequencing systems described above.

As now shown in the table 800c, the HLA-aware sequencing system 106 generated HLA genotyping calls with approximately 96-97% accuracy for TSO500, approximately 98% for WES, and approximately over 99% for an independent WGS cohort. In comparison to existing sequencing systems for TSO500 and WES described above, the HLA-aware sequencing system 106 exhibits an approximately 8-9% improvement in accuracy for TSO500 and an approximately 4-5% improvement in accuracy for WES.

As the tables 800a-800c therefore demonstrate, the HLA-aware sequencing system 106 improves HLA genotyping accuracy across different assays, including improved HLA genotyping accuracy in each of WGS, WES, and tumor gene panels. Such across-assay improvements in accuracy likewise enables clinicians to eliminate unnecessary additional assays and use fewer specialized computing devices to generate HLA genotype calls with improved accuracy.

Turning now to FIG. 9, this figure illustrates a flowchart of a series of acts 900 of genotyping one or more human leukocyte antigen (HLA) alleles from a genomic sample by using alignment-score-based filtering and read-support-equivalence grouping of nucleotide reads for genotype inference in accordance with one or more embodiments of the present disclosure. While FIG. 9 illustrates acts according to one embodiment, alternative embodiments may omit, add to, reorder, and/or modify any of the acts shown in FIG. 9. The acts of FIG. 9 can be performed as part of a method. Alternatively, a non-transitory computer readable storage medium can comprise instructions that, when executed by one or more processors, cause a computing device or a system to perform the acts depicted in FIG. 9. In still further embodiments, a system comprising at least one processor and a non-transitory computer readable medium comprising instructions that, when executed by one or more processors, cause the system to perform the acts of FIG. 9. In some cases, the at least one processor comprises a configurable processor and executing the at least one processor comprises configuring the configurable processor.

As shown in FIG. 9, the acts 900 include an act 902 of aligning human leukocyte antigen (HLA) nucleotide reads of a genomic sample with HLA-allele-reference sequences. In particular, in some embodiments, the act 902 includes aligning nucleotide reads corresponding to a human leukocyte antigen (HLA) genomic region of a genomic sample with HLA-allele-reference sequences.

As suggested above, in certain implementations, aligning the nucleotide reads with the HLA-allele-reference sequences comprises: grouping the HLA-allele-reference sequences into batches of HLA-allele-reference sequences; and aligning each nucleotide read with each HLA-allele-reference sequence from each batch of HLA-allele-reference sequences according to an order of an initial batch of HLA-allele-reference sequences to a final batch of HLA-allele-reference sequences.

As further shown in FIG. 9, the acts 900 include an act 904 of selecting a subset of nucleotide-read alignments based on alignment scores. In particular, in certain implementations, the act 904 includes selecting a subset of nucleotide-read alignments based on alignment scores for alignments between the nucleotide reads and the HLA-allele-reference sequences.

For example, in some cases, selecting the subset of nucleotide-read alignments comprises: determining local alignment scores for candidate alignments between each nucleotide read from a nucleotide read pair and each of the HLA-allele-reference sequences; and selecting, from among the candidate alignments, alignments of nucleotide read pairs and individual HLA-allele-references sequences that exhibit highest local alignment scores from among the local alignment scores.

As a further example, in certain implementations, selecting the subset of nucleotide-read alignments comprises: determining local alignment scores for candidate alignments between each nucleotide read from a nucleotide read pair and each of the HLA-allele-reference sequences; determining, from among the candidate alignments, that particular nucleotide-read alignments between nucleotide read pairs and individual HLA-allele-references sequences exhibit local alignment scores and nucleobase lengths satisfying a score-length-combination threshold; and selecting the particular nucleotide-read alignments that satisfy the score-length-combination threshold.

As further shown in FIG. 9, the acts 900 include an act 906 of grouping individual nucleotide reads into HLA equivalence classes. In particular, in certain implementations, the act 906 includes determining candidate HLA alleles for one or more loci within the HLA genomic region based on the HLA equivalence classes.

As further shown in FIG. 9, the acts 900 include an act 908 of determining candidate HLA alleles for one or more HLA loci based on the HLA equivalence classes. In particular, in certain implementations, the act 908 includes determining candidate HLA alleles for one or more loci within the HLA genomic region based on the HLA equivalence classes.

As suggested above, in some cases, determining the candidate HLA alleles comprises: performing a first stage of an expectation-maximization (EM) algorithm on the individual nucleotide reads grouped according to the HLA equivalence classes; and determining a first set of candidate allele probabilities that the genomic sample comprises a first set of candidate HLA alleles at two-field resolution. Relatedly, in certain embodiments, determining the candidate HLA alleles comprises: performing a second stage of the EM algorithm on individual nucleotide reads regrouped according to updated HLA equivalence classes based on the first set of candidate HLA alleles; and determining a second set of candidate allele probabilities that the genomic sample comprises a second set of candidate HLA alleles at full resolution.

As further shown in FIG. 9, the acts 900 include an act 910 of generating genotype calls that the genomic sample comprises one or more HLA alleles at the one or more HLA loci base on the candidate HLA alleles. In particular, in certain implementations, the act 910 includes generating genotype calls that the genomic sample comprises one or more HLA alleles at the one or more loci based on the candidate HLA alleles. For instance, in some cases, generating the genotype calls comprises generating a genotype call that the genomic sample comprises an HLA allele at full resolution.

As further suggested above, in some implementations, generating the genotype calls of HLA alleles comprises: determining, for an HLA locus, a candidate HLA allele at two-field resolution based on the first set of candidate allele probabilities from the first stage of the EM algorithm; determining that the candidate HLA allele at two-field resolution is among a top two candidate HLA alleles at full resolution exhibiting highest candidate allele probabilities from the second stage of the EM algorithm; and generating, for the HLA locus, a genotype call that the genomic sample comprises the candidate HLA allele at full resolution based on the candidate HLA allele being among the top two candidate HLA alleles at full resolution.

Relatedly, in certain embodiments, generating the genotype calls of HLA alleles comprises: determining, for an HLA locus, a candidate HLA allele at two-field resolution based on the first set of candidate allele probabilities from the first stage of the EM algorithm; determining that the candidate HLA allele at two-field resolution is among a threshold group of candidate HLA alleles at full resolution exhibiting highest candidate allele probabilities from the second stage of the EM algorithm; and generating, for the HLA locus, a genotype call that the genomic sample comprises the candidate HLA allele at full resolution based on the candidate HLA allele being among the threshold group of candidate HLA alleles at full resolution.

By contrast, in certain cases, generating the genotype calls of HLA alleles comprises: determining, for an HLA locus, a candidate HLA allele at two-field resolution based on the first set of candidate allele probabilities from the first stage of the EM algorithm; determining that the candidate HLA allele at two-field resolution is not among a top two candidate HLA alleles at full resolution exhibiting highest candidate allele probabilities from the second stage of the EM algorithm; and generating, for the HLA locus, a genotype call that the genomic sample comprises one of the top two candidate HLA alleles at full resolution based on the candidate HLA allele not being among the top two candidate HLA alleles at full resolution.

Relatedly, in certain embodiments, generating the genotype calls of HLA alleles comprises: determining, for an HLA locus, a candidate HLA allele at two-field resolution based on the first set of candidate allele probabilities from the first stage of the EM algorithm; determining that the candidate HLA allele at two-field resolution is not among a threshold group of candidate HLA alleles at full resolution exhibiting highest candidate allele probabilities from the second stage of the EM algorithm; and generating, for the HLA locus, a genotype call that the genomic sample comprises one of the top two candidate HLA alleles at full resolution based on the candidate HLA allele not being among the threshold group of candidate HLA alleles at full resolution.

Additionally, in one or more embodiments, generating the genotype calls comprises generating the genotype calls that the genomic sample comprises a pair of HLA-A alleles at an HLA-A locus, a pair of HLA-B alleles at an HLA-B locus, and a pair of HLA-C alleles at an HLA-C locus.

In addition or in the alternative to the acts 902-910, in some embodiments, the acts 900 include aligning a set of nucleotide reads with a reference genome; and extracting, from the set of nucleotide reads, the nucleotide reads corresponding to the HLA genomic region.

The methods described herein can be used in conjunction with a variety of nucleic acid sequencing techniques. Particularly applicable techniques are those wherein nucleic acids are attached at fixed locations in an array such that their relative positions do not change and wherein the array is repeatedly imaged. Embodiments in which images are obtained in different color channels, for example, coinciding with different labels used to distinguish one nucleobase type from another are particularly applicable. In some embodiments, the process to determine the nucleotide sequence of a target nucleic acid (i.e., a nucleic-acid polymer) can be an automated process. Preferred embodiments include sequencing-by-synthesis (SBS) techniques.

SBS techniques generally involve the enzymatic extension of a nascent nucleic acid strand through the iterative addition of nucleotides against a template strand. In traditional methods of SBS, a single nucleotide monomer may be provided to a target nucleotide in the presence of a polymerase in each delivery. However, in the methods described herein, more than one type of nucleotide monomer can be provided to a target nucleic acid in the presence of a polymerase in a delivery.

SBS can utilize nucleotide monomers that have a terminator moiety or those that lack any terminator moieties. Methods utilizing nucleotide monomers lacking terminators include, for example, pyrosequencing and sequencing using γ-phosphate-labeled nucleotides, as set forth in further detail below. In methods using nucleotide monomers lacking terminators, the number of nucleotides added in each cycle is generally variable and dependent upon the template sequence and the mode of nucleotide delivery. For SBS techniques that utilize nucleotide monomers having a terminator moiety, the terminator can be effectively irreversible under the sequencing conditions used as is the case for traditional Sanger sequencing which utilizes dideoxynucleotides, or the terminator can be reversible as is the case for sequencing methods developed by Solexa (now Illumina, Inc.).

SBS techniques can utilize nucleotide monomers that have a label moiety or those that lack a label moiety. Accordingly, incorporation events can be detected based on a characteristic of the label, such as fluorescence of the label; a characteristic of the nucleotide monomer such as molecular weight or charge; a byproduct of incorporation of the nucleotide, such as release of pyrophosphate; or the like. In embodiments, where two or more different nucleotides are present in a sequencing reagent, the different nucleotides can be distinguishable from each other, or alternatively, the two or more different labels can be the indistinguishable under the detection techniques being used. For example, the different nucleotides present in a sequencing reagent can have different labels and they can be distinguished using appropriate optics as exemplified by the sequencing methods developed by Solexa (now Illumina, Inc.).

Preferred embodiments include pyrosequencing techniques. Pyrosequencing detects the release of inorganic pyrophosphate (PPi) as particular nucleotides are incorporated into the nascent strand (Ronaghi, M., Karamohamed, S., Pettersson, B., Uhlen, M. and Nyren, P. (1996) "Real-time DNA sequencing using detection of pyrophosphate release." Analytical Biochemistry 242(1), 84-9; Ronaghi, M. (2001) "Pyrosequencing sheds light on DNA sequencing." Genome Res. 11(1), 3-11; Ronaghi, M., Uhlen, M. and Nyren, P. (1998) "A sequencing method based on real-time pyrophosphate." Science 281(5375), 363; U.S. Pat. No. 6,210,891; U.S. Pat. No. 6,258,568 and U.S. Pat. No. 6,274,320, the disclosures of which are incorporated herein by reference in their entireties). In pyrosequencing, released PPi can be detected by being immediately converted to adenosine triphosphate (ATP) by ATP sulfurylase, and the level of ATP generated is detected via luciferase-produced photons. The nucleic acids to be sequenced can be attached to features in an array and the array can be imaged to capture the chemiluminescent signals that are produced due to incorporation of a nucleotides at the features of the array. An image can be obtained after the array is treated with a particular nucleotide type (e.g., A, T, C or G). Images obtained after addition of each nucleotide type will differ with regard to which features in the array are detected. These differences in the image reflect the different sequence content of the features on the array. However, the relative locations of each feature will remain unchanged in the images. The images can be stored, processed and analyzed using the methods set forth herein. For example, images obtained after treatment of the array with each different nucleotide type can be handled in the same way as exemplified herein for images obtained from different detection channels for reversible terminator-based sequencing methods.

In another exemplary type of SBS, cycle sequencing is accomplished by stepwise addition of reversible terminator nucleotides containing, for example, a cleavable or photobleachable dye label as described, for example, in WO 04/018497 and U.S. Pat. No. 7,057,026, the disclosures of which are incorporated herein by reference. This approach is being commercialized by Solexa (now Illumina Inc.), and is also described in WO 91/06678 and WO 07/123,744, each of which is incorporated herein by reference. The availability of fluorescently-labeled terminators in which both the termination can be reversed and the fluorescent label cleaved facilitates efficient cyclic reversible termination (CRT) sequencing. Polymerases can also be co-engineered to efficiently incorporate and extend from these modified nucleotides.

Preferably in reversible terminator-based sequencing embodiments, the labels do not substantially inhibit extension under SBS reaction conditions. However, the detection labels can be removable, for example, by cleavage or degradation. Images can be captured following incorporation of labels into arrayed nucleic acid features. In particular embodiments, each cycle involves simultaneous delivery of four different nucleotide types to the array and each nucleotide type has a spectrally distinct label. Four images can then be obtained, each using a detection channel that is selective for one of the four different labels. Alternatively, different nucleotide types can be added sequentially and an image of the array can be obtained between each addition step. In such embodiments, each image will show nucleic acid features that have incorporated nucleotides of a particular type. Different features are present or absent in the different images due the different sequence content of each feature. However, the relative position of the features will remain unchanged in the images. Images obtained from such reversible terminator-SBS methods can be stored, processed and analyzed as set forth herein. Following the image capture step, labels can be removed and reversible terminator moieties can be removed for subsequent cycles of nucleotide addition and detection. Removal of the labels after they have been detected in a particular cycle and prior to a subsequent cycle can provide the advantage of reducing background signal and crosstalk between cycles. Examples of useful labels and removal methods are set forth below.

In particular embodiments some or all of the nucleotide monomers can include reversible terminators. In such embodiments, reversible terminators/cleavable fluors can include fluor linked to the ribose moiety via a 3' ester linkage (Metzker, Genome Res. 15:1767-1776 (2005), which is incorporated herein by reference). Other approaches have separated the terminator chemistry from the cleavage of the fluorescence label (Ruparel et al., Proc Natl Acad Sci USA 102: 5932-7 (2005), which is incorporated herein by reference in its entirety). Ruparel et al described the development of reversible terminators that used a small 3' allyl group to block extension, but could easily be deblocked by a short treatment with a palladium catalyst. The fluorophore was attached to the base via a photocleavable linker that could easily be cleaved by a 30 second exposure to long wavelength UV light. Thus, either disulfide reduction or photocleavage can be used as a cleavable linker. Another approach to reversible termination is the use of natural termination that ensues after placement of a bulky dye on a dNTP. The presence of a charged bulky dye on the dNTP can act as an effective terminator through steric and/or electrostatic hindrance. The presence of one incorporation event prevents further incorporations unless the dye is removed. Cleavage of the dye removes the fluor and effectively reverses the termination. Examples of modified nucleotides are also described in U.S. Pat. No. 7,427,673, and U.S. Pat. No. 7,057,026, the disclosures of which are incorporated herein by reference in their entireties.

Additional exemplary SBS systems and methods which can be utilized with the methods and systems described herein are described in U.S. Patent Application Publication No. 2007/0166705, U.S. Patent Application Publication No. 2006/0188901, U.S. Pat. No. 7,057,026, U.S. Patent Application Publication No. 2006/0240439, U.S. Patent Application Publication No. 2006/0281109, PCT Publication No. WO 05/065814, U.S. Patent Application Publication No. 2005/0100900, PCT Publication No. WO 06/064199, PCT Publication No. WO 07/010,251, U.S. Patent Application Publication No. 2012/0270305 and U.S. Patent Application Publication No. 2013/0260372, the disclosures of which are incorporated herein by reference in their entireties.

Some embodiments can utilize detection of four different nucleotides using fewer than four different labels. For example, SBS can be performed utilizing methods and systems described in the incorporated materials of U.S. Patent Application Publication No. 2013/0079232. As a first example, a pair of nucleotide types can be detected at the same wavelength, but distinguished based on a difference in intensity for one member of the pair compared to the other, or based on a change to one member of the pair (e.g. via chemical modification, photochemical modification or physical modification) that causes apparent signal to appear or disappear compared to the signal detected for the other member of the pair. As a second example, three of four different nucleotide types can be detected under particular conditions while a fourth nucleotide type lacks a label that is detectable under those conditions, or is minimally detected under those conditions (e.g., minimal detection due to background fluorescence, etc.). Incorporation of the first three nucleotide types into a nucleic acid can be determined based on presence of their respective signals and incorporation of the fourth nucleotide type into the nucleic acid can be determined based on absence or minimal detection of any signal. As a third example, one nucleotide type can include label(s) that are detected in two different channels, whereas other nucleotide types are detected in no more than one of the channels. The aforementioned three exemplary configurations are not considered mutually exclusive and can be used in various combinations. An exemplary embodiment that combines all three examples, is a fluorescent-based SBS method that uses a first nucleotide type that is detected in a first channel (e.g. dATP having a label that is detected in the first channel when excited by a first excitation wavelength), a second nucleotide type that is detected in a second channel (e.g. dCTP having a label that is detected in the second channel when excited by a second excitation wavelength), a third nucleotide type that is detected in both the first and the second channel (e.g. dTTP having at least one label that is detected in both channels when excited by the first and/or second excitation wavelength) and a fourth nucleotide type that lacks a label that is not, or minimally, detected in either channel (e.g. dGTP having no label).

Further, as described in the incorporated materials of U.S. Patent Application Publication No. 2013/0079232, sequencing data can be obtained using a single channel. In such so-called one-dye sequencing approaches, the first nucleotide type is labeled but the label is removed after the first image is generated, and the second nucleotide type is labeled only after a first image is generated. The third nucleotide type retains its label in both the first and second images, and the fourth nucleotide type remains unlabeled in both images.

Some embodiments can utilize sequencing by ligation techniques. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. As with other SBS methods, images can be obtained following treatment of an array of nucleic acid features with the labeled sequencing reagents. Each image will show nucleic acid features that have incorporated labels of a particular type. Different features are present or absent in the different images due the different sequence content of each feature, but the relative position of the features will remain unchanged in the images. Images obtained from ligation-based sequencing methods can be stored, processed and analyzed as set forth herein. Exemplary SBS systems and methods which can be utilized with the methods and systems described herein are described in U.S. Pat. No. 6,969,488, U.S. Pat. No. 6,172,218, and U.S. Pat. No. 6,306,597, the disclosures of which are incorporated herein by reference in their entireties.

Some embodiments can utilize nanopore sequencing (Deamer, D. W. & Akeson, M. "Nanopores and nucleic acids: prospects for ultrarapid sequencing." Trends Biotechnol. 18, 147-151 (2000); Deamer, D. and D. Branton, "Characterization of nucleic acids by nanopore analysis". Acc. Chem. Res. 35:817-825 (2002); Li, J., M. Gershow, D. Stein, E. Brandin, and J. A. Golovchenko, "DNA molecules and configurations in a solid-state nanopore microscope" Nat. Mater. 2:611-615 (2003), the disclosures of which are incorporated herein by reference in their entireties). In such embodiments, the target nucleic acid passes through a nanopore. The nanopore can be a synthetic pore or biological membrane protein, such as α-hemolysin. As the target nucleic acid passes through the nanopore, each base-pair can be identified by measuring fluctuations in the electrical conductance of the pore. (U.S. Pat. No. 7,001,792; Soni, G. V. & Meller, "A. Progress toward ultrafast DNA sequencing using solid-state nanopores." Clin. Chem. 53, 1996-2001 (2007); Healy, K. "Nanopore-based single-molecule DNA analysis." Nanomed. 2, 459-481 (2007); Cockroft, S. L., Chu, J., Amorin, M. & Ghadiri, M. R. "A single-molecule nanopore device detects DNA polymerase activity with single-nucleotide resolution." J. Am. Chem. Soc. 130, 818-820 (2008), the disclosures of which are incorporated herein by reference in their entireties). Data obtained from nanopore sequencing can be stored, processed and analyzed as set forth herein. In particular, the data can be treated as an image in accordance with the exemplary treatment of optical images and other images that is set forth herein.

Some embodiments can utilize methods involving the real-time monitoring of DNA polymerase activity. Nucleotide incorporations can be detected through fluorescence resonance energy transfer (FRET) interactions between a fluorophore-bearing polymerase and γ-phosphate-labeled nucleotides as described, for example, in U.S. Pat. No. 7,329,492 and U.S. Pat. No. 7,211,414 (each of which is incorporated herein by reference) or nucleotide incorporations can be detected with zero-mode waveguides as described, for example, in U.S. Pat. No. 7,315,019 (which is incorporated herein by reference) and using fluorescent nucleotide analogs and engineered polymerases as described, for example, in U.S. Pat. No. 7,405,281 and U.S. Patent Application Publication No. 2008/0108082 (each of which is incorporated herein by reference). The illumination can be restricted to a zeptoliter-scale volume around a surface-tethered polymerase such that incorporation of fluorescently labeled nucleotides can be observed with low background (Levene, M. J. et al. "Zero-mode waveguides for single-molecule analysis at high concentrations." Science 299, 682-686 (2003); Lundquist, P. M. et al. "Parallel confocal detection of single molecules in real time." Opt. Lett. 33, 1026-1028 (2008); Korlach, J. et al. "Selective aluminum passivation for targeted immobilization of single DNA polymerase molecules in zero-mode waveguide nano structures." Proc. Natl. Acad. Sci. USA 105, 1176-1181 (2008), the disclosures of which are incorporated herein by reference in their entireties). Images obtained from such methods can be stored, processed and analyzed as set forth herein.

Some SBS embodiments include detection of a proton released upon incorporation of a nucleotide into an extension product. For example, sequencing based on detection of released protons can use an electrical detector and associated techniques that are commercially available from Ion Torrent (Guilford, CT, a Life Technologies subsidiary) or sequencing methods and systems described in US 2009/0026082 A1; US 2009/0127589 A1; US 2010/0137143 A1; or US 2010/0282617 A1, each of which is incorporated herein by reference. Methods set forth herein for amplifying target nucleic acids using kinetic exclusion can be readily applied to substrates used for detecting protons. More specifically, methods set forth herein can be used to produce clonal populations of amplicons that are used to detect protons.

The above SBS methods can be advantageously carried out in multiplex formats such that multiple different target nucleic acids are manipulated simultaneously. In particular embodiments, different target nucleic acids can be treated in a common reaction vessel or on a surface of a particular substrate. This allows convenient delivery of sequencing reagents, removal of unreacted reagents and detection of incorporation events in a multiplex manner. In embodiments using surface-bound target nucleic acids, the target nucleic acids can be in an array format. In an array format, the target nucleic acids can be typically bound to a surface in a spatially distinguishable manner. The target nucleic acids can be bound by direct covalent attachment, attachment to a bead or other particle or binding to a polymerase or other molecule that is attached to the surface. The array can include a single copy of a target nucleic acid at each site (also referred to as a feature) or multiple copies having the same sequence can be present at each site or feature. Multiple copies can be produced by amplification methods such as, bridge amplification or emulsion PCR as described in further detail below.

The methods set forth herein can use arrays having features at any of a variety of densities including, for example, at least about 10 features/cm2, 100 features/cm2, 500 features/cm2, 1,000 features/cm2, 5,000 features/cm2, 10,000 features/cm2, 50,000 features/cm2, 100,000 features/cm2, 1,000,000 features/cm2, 5,000,000 features/cm2, or higher.

An advantage of the methods set forth herein is that they provide for rapid and efficient detection of a plurality of target nucleic acid in parallel. Accordingly the present disclosure provides integrated systems capable of preparing and detecting nucleic acids using techniques known in the art such as those exemplified above. Thus, an integrated system of the present disclosure can include fluidic components capable of delivering amplification reagents and/or sequencing reagents to one or more immobilized DNA fragments, the system comprising components such as pumps, valves, reservoirs, fluidic lines and the like. A flow cell can be configured and/or used in an integrated system for detection of target nucleic acids. Exemplary flow cells are described, for example, in US 2010/0111768 A1 and US Ser. No. 13/273,666, each of which is incorporated herein by reference. As exemplified for flow cells, one or more of the fluidic components of an integrated system can be used for an amplification method and for a detection method. Taking a nucleic acid sequencing embodiment as an example, one or more of the fluidic components of an integrated system can be used for an amplification method set forth herein and for the delivery of sequencing reagents in a sequencing method such as those exemplified above. Alternatively, an integrated system can include separate fluidic systems to carry out amplification methods and to carry out detection methods. Examples of integrated sequencing systems that are capable of creating amplified nucleic acids and also determining the sequence of the nucleic acids include, without limitation, the MiSeqTM platform (Illumina, Inc., San Diego, CA) and devices described in US Ser. No. 13/273,666, which is incorporated herein by reference.

The sequencing system described above sequences nucleic-acid polymers present in samples received by a sequencing device. As defined herein, "sample" and its derivatives, is used in its broadest sense and includes any specimen, culture and the like that is suspected of including a target. In some embodiments, the sample comprises DNA, RNA, PNA, LNA, chimeric or hybrid forms of nucleic acids. The sample can include any biological, clinical, surgical, agricultural, atmospheric or aquatic-based specimen containing one or more nucleic acids. The term also includes any isolated nucleic acid sample such a genomic DNA, fresh-frozen or formalin-fixed paraffin-embedded nucleic acid specimen. It is also envisioned that the sample can be from a single individual, a collection of nucleic acid samples from genetically related members, nucleic acid samples from genetically unrelated members, nucleic acid samples (matched) from a single individual such as a tumor sample and normal tissue sample, or sample from a single source that contains two distinct forms of genetic material such as maternal and fetal DNA obtained from a maternal subject, or the presence of contaminating bacterial DNA in a sample that contains plant or animal DNA. In some embodiments, the source of nucleic acid material can include nucleic acids obtained from a newborn, for example as typically used for newborn screening.

The nucleic acid sample can include high molecular weight material such as genomic DNA (gDNA). The sample can include low molecular weight material such as nucleic acid molecules obtained from FFPE or archived DNA samples. In another embodiment, low molecular weight material includes enzymatically or mechanically fragmented DNA. The sample can include cell-free circulating DNA. In some embodiments, the sample can include nucleic acid molecules obtained from biopsies, tumors, scrapings, swabs, blood, mucus, urine, plasma, semen, hair, laser capture micro-dissections, surgical resections, and other clinical or laboratory obtained samples. In some embodiments, the sample can be an epidemiological, agricultural, forensic or pathogenic sample. In some embodiments, the sample can include nucleic acid molecules obtained from an animal such as a human or mammalian source. In another embodiment, the sample can include nucleic acid molecules obtained from a non-mammalian source such as a plant, bacteria, virus or fungus. In some embodiments, the source of the nucleic acid molecules may be an archived or extinct sample or species.

Further, the methods and compositions disclosed herein may be useful to amplify a nucleic acid sample having low-quality nucleic acid molecules, such as degraded and/or fragmented genomic DNA from a forensic sample. In one embodiment, forensic samples can include nucleic acids obtained from a crime scene, nucleic acids obtained from a missing persons DNA database, nucleic acids obtained from a laboratory associated with a forensic investigation or include forensic samples obtained by law enforcement agencies, one or more military services or any such personnel. The nucleic acid sample may be a purified sample or a crude DNA containing lysate, for example derived from a buccal swab, paper, fabric or other substrate that may be impregnated with saliva, blood, or other bodily fluids. As such, in some embodiments, the nucleic acid sample may comprise low amounts of, or fragmented portions of DNA, such as genomic DNA. In some embodiments, target sequences can be present in one or more bodily fluids including but not limited to, blood, sputum, plasma, semen, urine and serum. In some embodiments, target sequences can be obtained from hair, skin, tissue samples, autopsy or remains of a victim. In some embodiments, nucleic acids including one or more target sequences can be obtained from a deceased animal or human. In some embodiments, target sequences can include nucleic acids obtained from non-human DNA such a microbial, plant or entomological DNA. In some embodiments, target sequences or amplified target sequences are directed to purposes of human identification. In some embodiments, the disclosure relates generally to methods for identifying characteristics of a forensic sample. In some embodiments, the disclosure relates generally to human identification methods using one or more target specific primers disclosed herein or one or more target specific primers designed using the primer design criteria outlined herein. In one embodiment, a forensic or human identification sample containing at least one target sequence can be amplified using any one or more of the target-specific primers disclosed herein or using the primer criteria outlined herein.

The components of the HLA-aware sequencing system 106 can include software, hardware, or both. For example, the components of the HLA-aware sequencing system 106 can include one or more instructions stored on a computer-readable storage medium and executable by processors of one or more computing devices (e.g., the client device 114). When executed by the one or more processors, the computer-executable instructions of the HLA-aware sequencing system 106 can cause the computing devices to perform the bubble detection methods described herein. Alternatively, the components of the HLA-aware sequencing system 106 can comprise hardware, such as special purpose processing devices to perform a certain function or group of functions. Additionally, or alternatively, the components of the HLA-aware sequencing system 106 can include a combination of computer-executable instructions and hardware.

Furthermore, the components of the HLA-aware sequencing system 106 performing the functions described herein with respect to the HLA-aware sequencing system 106 may, for example, be implemented as part of a stand-alone application, as a module of an application, as a plug-in for applications, as a library function or functions that may be called by other applications, and/or as a cloud-computing model. Thus, components of the HLA-aware sequencing system 106 may be implemented as part of a stand-alone application on a personal computing device or a mobile device. Additionally, or alternatively, the components of the HLA-aware sequencing system 106 may be implemented in any application that provides sequencing services including, but not limited to Illumina BaseSpace, Illumina DRAGEN, Illumina NextSeq, Illumina TruSeq, or Illumina TruSight software. "Illumina," "BaseSpace," "DRAGEN," "NextSeq," "TruSeq," and "TruSight," are either registered trademarks or trademarks of Illumina, Inc. in the United States and/or other countries.

Embodiments of the present disclosure may comprise or utilize a special purpose or general-purpose computer including computer hardware, such as, for example, one or more processors and system memory, as discussed in greater detail below. Embodiments within the scope of the present disclosure also include physical and other computer-readable media for carrying or storing computer-executable instructions and/or data structures. In particular, one or more of the processes described herein may be implemented at least in part as instructions embodied in a non-transitory computer-readable medium and executable by one or more computing devices (e.g., any of the media content access devices described herein). In general, a processor (e.g., a microprocessor) receives instructions, from a non-transitory computer-readable medium, (e.g., a memory, etc.), and executes those instructions, thereby performing one or more processes, including one or more of the processes described herein.

Computer-readable media can be any available media that can be accessed by a general purpose or special purpose computer system. Computer-readable media that store computer-executable instructions are non-transitory computer-readable storage media (devices). Computer-readable media that carry computer-executable instructions are transmission media. Thus, by way of example, and not limitation, embodiments of the disclosure can comprise at least two distinctly different kinds of computer-readable media: non-transitory computer-readable storage media (devices) and transmission media.

Non-transitory computer-readable storage media (devices) includes RAM, ROM, EEPROM, CD-ROM, solid state drives (SSDs) (e.g., based on RAM), Flash memory, phase-change memory (PCM), other types of memory, other optical disk storage, magnetic disk storage or other magnetic storage devices, or any other medium which can be used to store desired program code means in the form of computer-executable instructions or data structures and which can be accessed by a general purpose or special purpose computer.

A "network" is defined as one or more data links that enable the transport of electronic data between computer systems and/or modules and/or other electronic devices. When information is transferred or provided over a network or another communications connection (either hardwired, wireless, or a combination of hardwired or wireless) to a computer, the computer properly views the connection as a transmission medium. Transmissions media can include a network and/or data links which can be used to carry desired program code means in the form of computer-executable instructions or data structures and which can be accessed by a general purpose or special purpose computer. Combinations of the above should also be included within the scope of computer-readable media.

Further, upon reaching various computer system components, program code means in the form of computer-executable instructions or data structures can be transferred automatically from transmission media to non-transitory computer-readable storage media (devices) (or vice versa). For example, computer-executable instructions or data structures received over a network or data link can be buffered in RAM within a network interface module (e.g., a NIC), and then eventually transferred to computer system RAM and/or to less volatile computer storage media (devices) at a computer system. Thus, it should be understood that non-transitory computer-readable storage media (devices) can be included in computer system components that also (or even primarily) utilize transmission media.

Computer-executable instructions comprise, for example, instructions and data which, when executed at a processor, cause a general-purpose computer, special purpose computer, or special purpose processing device to perform a certain function or group of functions. In some embodiments, computer-executable instructions are executed on a general-purpose computer to turn the general-purpose computer into a special purpose computer implementing elements of the disclosure. The computer executable instructions may be, for example, binaries, intermediate format instructions such as assembly language, or even source code. Although the subject matter has been described in language specific to structural features and/or methodological acts, it is to be understood that the subject matter defined in the appended claims is not necessarily limited to the described features or acts described above. Rather, the described features and acts are disclosed as example forms of implementing the claims.

Those skilled in the art will appreciate that the disclosure may be practiced in network computing environments with many types of computer system configurations, including, personal computers, desktop computers, laptop computers, message processors, hand-held devices, multiprocessor systems, microprocessor-based or programmable consumer electronics, network PCs, minicomputers, mainframe computers, mobile telephones, PDAs, tablets, pagers, routers, switches, and the like. The disclosure may also be practiced in distributed system environments where local and remote computer systems, which are linked (either by hardwired data links, wireless data links, or by a combination of hardwired and wireless data links) through a network, both perform tasks. In a distributed system environment, program modules may be located in both local and remote memory storage devices.

Embodiments of the present disclosure can also be implemented in cloud computing environments. In this description, "cloud computing" is defined as a model for enabling on-demand network access to a shared pool of configurable computing resources. For example, cloud computing can be employed in the marketplace to offer ubiquitous and convenient on-demand access to the shared pool of configurable computing resources. The shared pool of configurable computing resources can be rapidly provisioned via virtualization and released with low management effort or service provider interaction, and then scaled accordingly.

A cloud-computing model can be composed of various characteristics such as, for example, on-demand self-service, broad network access, resource pooling, rapid elasticity, measured service, and so forth. A cloud-computing model can also expose various service models, such as, for example, Software as a Service (SaaS), Platform as a Service (PaaS), and Infrastructure as a Service (IaaS). A cloud-computing model can also be deployed using different deployment models such as private cloud, community cloud, public cloud, hybrid cloud, and so forth. In this description and in the claims, a "cloud-computing environment" is an environment in which cloud computing is employed.

FIG. 10 illustrates a block diagram of a computing device 1000 that may be configured to perform one or more of the processes described above. One will appreciate that one or more computing devices such as the computing device 1000 may implement the HLA-aware sequencing system 106 and the HLA-aware sequencing system 106. As shown by FIG. 10, the computing device 1000 can comprise a processor 1002, a memory 1004, a storage device 1006, an I/O interface 1008, and a communication interface 1010, which may be communicatively coupled by way of a communication infrastructure 1012. In certain embodiments, the computing device 1000 can include fewer or more components than those shown in FIG. 10. The following paragraphs describe components of the computing device 1000 shown in FIG. 10 in additional detail.

In one or more embodiments, the processor 1002 includes hardware for executing instructions, such as those making up a computer program. As an example, and not by way of limitation, to execute instructions for dynamically modifying workflows, the processor 1002 may retrieve (or fetch) the instructions from an internal register, an internal cache, the memory 1004, or the storage device 1006 and decode and execute them. The memory 1004 may be a volatile or nonvolatile memory used for storing data, metadata, and programs for execution by the processor(s). The storage device 1006 includes storage, such as a hard disk, flash disk drive, or other digital storage device, for storing data or instructions for performing the methods described herein.

The I/O interface 1008 allows a user to provide input to, receive output from, and otherwise transfer data to and receive data from computing device 1000. The I/O interface 1008 may include a mouse, a keypad or a keyboard, a touch screen, a camera, an optical scanner, network interface, modem, other known I/O devices or a combination of such I/O interfaces. The I/O interface 1008 may include one or more devices for presenting output to a user, including, but not limited to, a graphics engine, a display (e.g., a display screen), one or more output drivers (e.g., display drivers), one or more audio speakers, and one or more audio drivers. In certain embodiments, the I/O interface 1008 is configured to provide graphical data to a display for presentation to a user. The graphical data may be representative of one or more graphical user interfaces and/or any other graphical content as may serve a particular implementation.

The communication interface 1010 can include hardware, software, or both. In any event, the communication interface 1010 can provide one or more interfaces for communication (such as, for example, packet-based communication) between the computing device 1000 and one or more other computing devices or networks. As an example, and not by way of limitation, the communication interface 1010 may include a network interface controller (NIC) or network adapter for communicating with an Ethernet or other wire-based network or a wireless NIC (WNIC) or wireless adapter for communicating with a wireless network, such as a WI-FI.

Additionally, the communication interface 1010 may facilitate communications with various types of wired or wireless networks. The communication interface 1010 may also facilitate communications using various communication protocols. The communication infrastructure 1012 may also include hardware, software, or both that couples components of the computing device 1000 to each other. For example, the communication interface 1010 may use one or more networks and/or protocols to enable a plurality of computing devices connected by a particular infrastructure to communicate with each other to perform one or more aspects of the processes described herein. To illustrate, the sequencing process can allow a plurality of devices (e.g., a client device, sequencing device, and server device(s)) to exchange information such as sequencing data and error notifications.

In the foregoing specification, the present disclosure has been described with reference to specific exemplary embodiments thereof. Various embodiments and aspects of the present disclosure(s) are described with reference to details discussed herein, and the accompanying drawings illustrate the various embodiments. The description above and drawings are illustrative of the disclosure and are not to be construed as limiting the disclosure. Numerous specific details are described to provide a thorough understanding of various embodiments of the present disclosure.

The present disclosure may be embodied in other specific forms without departing from its spirit or essential characteristics. The described embodiments are to be considered in all respects only as illustrative and not restrictive. For example, the methods described herein may be performed with less or more steps/acts or the steps/acts may be performed in differing orders. Additionally, the steps/acts described herein may be repeated or performed in parallel with one another or in parallel with different instances of the same or similar steps/acts. The scope of the present application is, therefore, indicated by the appended claims rather than by the foregoing description. All changes that come within the meaning and range of equivalency of the claims are to be embraced within their scope.

Aspects can be understood from the following numbered paragraphs
1. A computer-implemented method comprising:
   aligning nucleotide reads corresponding to a human leukocyte antigen (HLA) genomic region of a genomic sample with HLA-allele-reference sequences;
   selecting a subset of nucleotide-read alignments based on alignment scores for alignments between the nucleotide reads and the HLA-allele-reference sequences;
   grouping individual nucleotide reads corresponding to the subset of nucleotide-read alignments into HLA equivalence classes;
   determining candidate HLA alleles for one or more loci within the HLA genomic region based on the HLA equivalence classes; and
   generating genotype calls that the genomic sample comprises one or more HLA alleles at the one or more loci based on the candidate HLA alleles.
2. The computer-implemented method of paragraph 1, wherein selecting the subset of nucleotide-read alignments comprises:
   determining local alignment scores for candidate alignments between each nucleotide read from a nucleotide read pair and each of the HLA-allele-reference sequences; and
   selecting, from among the candidate alignments, alignments of nucleotide read pairs and individual HLA-allele-references sequences that exhibit highest local alignment scores from among the local alignment scores.
3. The computer-implemented method of paragraph 1, wherein selecting the subset of nucleotide-read alignments comprises:
   determining local alignment scores for candidate alignments between each nucleotide read from a nucleotide read pair and each of the HLA-allele-reference sequences;
   determining, from among the candidate alignments, that particular nucleotide-read alignments between nucleotide read pairs and individual HLA-allele-references sequences exhibit local alignment scores and nucleobase lengths satisfying a score-length-combination threshold; and
   selecting the particular nucleotide-read alignments that satisfy the score-length-combination threshold.
4. The computer-implemented method of paragraph 1, wherein generating the genotype calls comprises generating a genotype call that the genomic sample comprises an HLA allele at full resolution.
5. The computer-implemented method of paragraph 1, wherein determining the candidate HLA alleles comprises:
   performing a first stage of an expectation-maximization (EM) algorithm on the individual nucleotide reads grouped according to the HLA equivalence classes; and
   determining a first set of candidate allele probabilities that the genomic sample comprises a first set of candidate HLA alleles at two-field resolution.
6. The computer-implemented method of paragraph 5, wherein determining the candidate HLA alleles comprises:
   performing a second stage of the EM algorithm on individual nucleotide reads regrouped according to updated HLA equivalence classes based on the first set of candidate HLA alleles; and
   determining a second set of candidate allele probabilities that the genomic sample comprises a second set of candidate HLA alleles at full resolution.
7. The computer-implemented method of paragraph 6, wherein generating the genotype calls of HLA alleles comprises:
   determining, for an HLA locus, a candidate HLA allele at two-field resolution based on the first set of candidate allele probabilities from the first stage of the EM algorithm;
   determining that the candidate HLA allele at two-field resolution is among a top two candidate HLA alleles at full resolution exhibiting highest candidate allele probabilities from the second stage of the EM algorithm; and
   generating, for the HLA locus, a genotype call that the genomic sample comprises the candidate HLA allele at full resolution based on the candidate HLA allele being among the top two candidate HLA alleles at full resolution.
8. The computer-implemented method of paragraph 6, wherein generating the genotype calls of HLA alleles comprises:
   determining, for an HLA locus, a candidate HLA allele at two-field resolution based on the first set of candidate allele probabilities from the first stage of the EM algorithm;
   determining that the candidate HLA allele at two-field resolution is not among a top two candidate HLA alleles at full resolution exhibiting highest candidate allele probabilities from the second stage of the EM algorithm; and
   generating, for the HLA locus, a genotype call that the genomic sample comprises one of the top two candidate HLA alleles at full resolution based on the candidate HLA allele not being among the top two candidate HLA alleles at full resolution.
9. The computer-implemented method of paragraph 1, wherein aligning the nucleotide reads with the HLA-allele-reference sequences comprises:
   grouping the HLA-allele-reference sequences into batches of HLA-allele-reference sequences; and
   aligning each nucleotide read with each HLA-allele-reference sequence from each batch of HLA-allele-reference sequences according to an order of an initial batch of HLA-allele-reference sequences to a final batch of HLA-allele-reference sequences.
10. The computer-implemented method of paragraph 1, wherein generating the genotype calls comprises generating the genotype calls that the genomic sample comprises a pair of HLA-A alleles at an HLA-A locus, a pair of HLA-B alleles at an HLA-B locus, and a pair of HLA-C alleles at an HLA-C locus.
11. The computer-implemented method of paragraph 1, further comprising:
   aligning a set of nucleotide reads with a reference genome; and
   extracting, from the set of nucleotide reads, the nucleotide reads corresponding to the HLA genomic region.
12. A non-transitory computer-readable medium comprising instructions that, when executed by at least one processor, cause a computing device to:
   align nucleotide reads corresponding to a human leukocyte antigen (HLA) genomic region of a genomic sample with HLA-allele-reference sequences;
   select a subset of nucleotide-read alignments based on alignment scores for alignments between the nucleotide reads and the HLA-allele-reference sequences;
   group individual nucleotide reads corresponding to the subset of nucleotide-read alignments into HLA equivalence classes;
   determine candidate HLA alleles for one or more loci within the HLA genomic region based on the HLA equivalence classes; and
   generate genotype calls that the genomic sample comprises one or more HLA alleles at the one or more loci based on the candidate HLA alleles.
13. The non-transitory computer-readable medium of paragraph 12, further comprising instructions that, when executed by the at least one processor, cause the computing device to select the subset of nucleotide-read alignments by:
   determining local alignment scores for candidate alignments between each nucleotide read from a nucleotide read pair and each of the HLA-allele-reference sequences; and
   selecting, from among the candidate alignments, alignments of nucleotide read pairs and individual HLA-allele-references sequences that exhibit highest local alignment scores from among the local alignment scores.
14. The non-transitory computer-readable medium of paragraph 12, further comprising instructions that, when executed by the at least one processor, cause the computing device to select the subset of nucleotide-read alignments by:
   determining local alignment scores for candidate alignments between each nucleotide read from a nucleotide read pair and each of the HLA-allele-reference sequences;
   determining, from among the candidate alignments, that particular nucleotide-read alignments between nucleotide read pairs and individual HLA-allele-references sequences exhibit local alignment scores and nucleobase lengths satisfying a score-length-combination threshold; and
   selecting the particular nucleotide-read alignments that satisfy the score-length-combination threshold.
15. The non-transitory computer-readable medium of paragraph 12, further comprising instructions that, when executed by the at least one processor, cause the computing device to generate the genotype calls by generating a genotype call that the genomic sample comprises an HLA allele at full resolution.
16. The non-transitory computer-readable medium of paragraph 12, further comprising instructions that, when executed by the at least one processor, cause the computing device to determine the candidate HLA alleles by:
   performing a first stage of an expectation-maximization (EM) algorithm on the individual nucleotide reads grouped according to the HLA equivalence classes; and
   determining a first set of candidate allele probabilities that the genomic sample comprises a first set of candidate HLA alleles at two-field resolution.
17. The non-transitory computer-readable medium of paragraph 16, further comprising instructions that, when executed by the at least one processor, cause the computing device to determine the candidate HLA alleles by:
   performing a second stage of the EM algorithm on individual nucleotide reads regrouped according to updated HLA equivalence classes based on the first set of candidate HLA alleles; and
   determining a second set of candidate allele probabilities that the genomic sample comprises a second set of candidate HLA alleles at full resolution.
18. The non-transitory computer-readable medium of paragraph 17, further comprising instructions that, when executed by the at least one processor, cause the computing device to generate the genotype calls of HLA alleles by:
   determining, for an HLA locus, a candidate HLA allele at two-field resolution based on the first set of candidate allele probabilities from the first stage of the EM algorithm;
   determining that the candidate HLA allele at two-field resolution is among a threshold group of candidate HLA alleles at full resolution exhibiting highest candidate allele probabilities from the second stage of the EM algorithm; and
   generating, for the HLA locus, a genotype call that the genomic sample comprises the candidate HLA allele at full resolution based on the candidate HLA allele being among the threshold group of candidate HLA alleles at full resolution.
19. The non-transitory computer-readable medium of paragraph 17, further comprising instructions that, when executed by the at least one processor, cause the computing device to generate the genotype calls of HLA alleles by:
   determining, for an HLA locus, a candidate HLA allele at two-field resolution based on the first set of candidate allele probabilities from the first stage of the EM algorithm;
   determining that the candidate HLA allele at two-field resolution is not among a threshold group of candidate HLA alleles at full resolution exhibiting highest candidate allele probabilities from the second stage of the EM algorithm; and
   generating, for the HLA locus, a genotype call that the genomic sample comprises one of the threshold group of HLA alleles at full resolution based on the candidate HLA allele not being among the threshold group of candidate HLA alleles at full resolution.
20. A system comprising:
   at least one processor; and
   a non-transitory computer readable medium comprising instructions that, when executed by the at least one processor, cause the system to:
      align nucleotide reads corresponding to a human leukocyte antigen (HLA) genomic region of a genomic sample with HLA-allele-reference sequences;
      select a subset of nucleotide-read alignments based on alignment scores for alignments between the nucleotide reads and the HLA-allele-reference sequences;
      group individual nucleotide reads corresponding to the subset of nucleotide-read alignments into HLA equivalence classes;
      determine candidate HLA alleles for one or more loci within the HLA genomic region based on the HLA equivalence classes; and
      generate genotype calls that the genomic sample comprises one or more HLA alleles at the one or more loci based on the candidate HLA alleles.
21. The system of paragraph 20, further comprising instructions that, when executed by the at least one processor, cause the system to select the subset of nucleotide-read alignments by:
   determining local alignment scores for candidate alignments between each nucleotide read from a nucleotide read pair and each of the HLA-allele-reference sequences; and
   selecting, from among the candidate alignments, alignments of nucleotide read pairs and individual HLA-allele-references sequences that exhibit highest local alignment scores from among the local alignment scores.
22. The system of paragraph 20, further comprising instructions that, when executed by the at least one processor, cause the system to select the subset of nucleotide-read alignments by:
   determining local alignment scores for candidate alignments between each nucleotide read from a nucleotide read pair and each of the HLA-allele-reference sequences;
   determining, from among the candidate alignments, that particular nucleotide-read alignments between nucleotide read pairs and individual HLA-allele-references sequences exhibit local alignment scores and nucleobase lengths satisfying a score-length-combination threshold; and
   selecting the particular nucleotide-read alignments that satisfy the score-length-combination threshold.
23. The system of paragraph 20, further comprising instructions that, when executed by the at least one processor, cause the system to generate the genotype calls by generating a genotype call that the genomic sample comprises an HLA allele at full resolution.
24. The system of paragraph 20, further comprising instructions that, when executed by the at least one processor, cause the system to determine the candidate HLA alleles by:
   performing a first stage of an expectation-maximization (EM) algorithm on the individual nucleotide reads grouped according to the HLA equivalence classes; and
   determining a first set of candidate allele probabilities that the genomic sample comprises a first set of candidate HLA alleles at two-field resolution.
25. The system of paragraph 24, further comprising instructions that, when executed by the at least one processor, cause the system to determine the candidate HLA alleles by:
   performing a second stage of the EM algorithm on individual nucleotide reads regrouped according to updated HLA equivalence classes based on the first set of candidate HLA alleles; and
   determining a second set of candidate allele probabilities that the genomic sample comprises a second set of candidate HLA alleles at full resolution.
26. The system of paragraph 20, further comprising instructions that, when executed by the at least one processor, cause the system to align the nucleotide reads with the HLA-allele-reference sequences by:
   grouping the HLA-allele-reference sequences into batches of HLA-allele-reference sequences; and
   aligning each nucleotide read with each HLA-allele-reference sequence from each batch of HLA-allele-reference sequences according to an order of an initial batch of HLA-allele-reference sequences to a final batch of HLA-allele-reference sequences.
27. The system of paragraph 20, further comprising instructions that, when executed by the at least one processor, cause the system to generate the genotype calls by generating the genotype calls that the genomic sample comprises a pair of HLA-A alleles at an HLA-A locus, a pair of HLA-B alleles at an HLA-B locus, and a pair of HLA-C alleles at an HLA-C locus.
28. The system of paragraph 20, further comprising instructions that, when executed by the at least one processor, cause the system to:
   align a set of nucleotide reads with a reference genome; and
   extract, from the set of nucleotide reads, the nucleotide reads corresponding to the HLA genomic region.

## Claims

1. A computer-implemented method comprising:
aligning nucleotide reads corresponding to a human leukocyte antigen (HLA) genomic region of a genomic sample with HLA-allele-reference sequences;
selecting a subset of nucleotide-read alignments based on alignment scores for alignments between the nucleotide reads and the HLA-allele-reference sequences;
grouping individual nucleotide reads corresponding to the subset of nucleotide-read alignments into HLA equivalence classes;
determining candidate HLA alleles for one or more loci within the HLA genomic region based on the HLA equivalence classes; and
generating genotype calls that the genomic sample comprises one or more HLA alleles at the one or more loci based on the candidate HLA alleles.

2. The computer-implemented method of claim 1, wherein selecting the subset of nucleotide-read alignments comprises:
determining local alignment scores for candidate alignments between each nucleotide read from a nucleotide read pair and each of the HLA-allele-reference sequences; and
selecting, from among the candidate alignments, alignments of nucleotide read pairs and individual HLA-allele-references sequences that exhibit highest local alignment scores from among the local alignment scores.

3. The computer-implemented method of claim 1 or 2, wherein selecting the subset of nucleotide-read alignments comprises:
determining local alignment scores for candidate alignments between each nucleotide read from a nucleotide read pair and each of the HLA-allele-reference sequences;
determining, from among the candidate alignments, that particular nucleotide-read alignments between nucleotide read pairs and individual HLA-allele-references sequences exhibit local alignment scores and nucleobase lengths satisfying a score-length-combination threshold; and
selecting the particular nucleotide-read alignments that satisfy the score-length-combination threshold.

4. The computer-implemented method of any one of claims 1-3, wherein generating the genotype calls comprises generating a genotype call that the genomic sample comprises an HLA allele at full resolution.

5. The computer-implemented method of any one of claims 1-4, wherein determining the candidate HLA alleles comprises:
performing a first stage of an expectation-maximization (EM) algorithm on the individual nucleotide reads grouped according to the HLA equivalence classes; and
determining a first set of candidate allele probabilities that the genomic sample comprises a first set of candidate HLA alleles at two-field resolution.

6. The computer-implemented method of any one of claims 1- 5, wherein determining the candidate HLA alleles comprises:
performing a second stage of the EM algorithm on individual nucleotide reads regrouped according to updated HLA equivalence classes based on the first set of candidate HLA alleles; and
determining a second set of candidate allele probabilities that the genomic sample comprises a second set of candidate HLA alleles at full resolution.

7. The computer-implemented method of any one of claims 1- 6, wherein generating the genotype calls of HLA alleles comprises:
determining, for an HLA locus, a candidate HLA allele at two-field resolution based on the first set of candidate allele probabilities from the first stage of the EM algorithm;
determining that the candidate HLA allele at two-field resolution is among a top two candidate HLA alleles at full resolution exhibiting highest candidate allele probabilities from the second stage of the EM algorithm; and
generating, for the HLA locus, a genotype call that the genomic sample comprises the candidate HLA allele at full resolution based on the candidate HLA allele being among the top two candidate HLA alleles at full resolution.

8. The computer-implemented method of any one of claims 1-6, wherein generating the genotype calls of HLA alleles comprises:
determining, for an HLA locus, a candidate HLA allele at two-field resolution based on the first set of candidate allele probabilities from the first stage of the EM algorithm;
determining that the candidate HLA allele at two-field resolution is not among a top two candidate HLA alleles at full resolution exhibiting highest candidate allele probabilities from the second stage of the EM algorithm; and
generating, for the HLA locus, a genotype call that the genomic sample comprises one of the top two candidate HLA alleles at full resolution based on the candidate HLA allele not being among the top two candidate HLA alleles at full resolution.

9. The computer-implemented method of any one of claims 1-8, wherein aligning the nucleotide reads with the HLA-allele-reference sequences comprises:
grouping the HLA-allele-reference sequences into batches of HLA-allele-reference sequences; and
aligning each nucleotide read with each HLA-allele-reference sequence from each batch of HLA-allele-reference sequences according to an order of an initial batch of HLA-allele-reference sequences to a final batch of HLA-allele-reference sequences.

10. The computer-implemented method of any one of claims 1-9, wherein generating the genotype calls comprises generating the genotype calls that the genomic sample comprises a pair of HLA-A alleles at an HLA-A locus, a pair of HLA-B alleles at an HLA-B locus, and a pair of HLA-C alleles at an HLA-C locus.

11. The computer-implemented method of any one of claims 1-11, further comprising:
aligning a set of nucleotide reads with a reference genome; and
extracting, from the set of nucleotide reads, the nucleotide reads corresponding to the HLA genomic region.

12. The computer-implemented method of any one of claims 1-11, wherein generating the genotype calls comprises generating a genotype call that the genomic sample comprises an HLA allele for an intron variant, and/ or wherein generating the genotype calls comprises generating the genotype calls for multiple loci within the HLA genomic region.

13. The computer-implemented method of any one of claims 1-12, wherein the nucleotide reads corresponding to the HLA genomic region of the genomic sample are extracted for whole genome sequencing (WGS), whole exome sequencing (WES), or a tumor gene panel.

14. A system comprising at least one processor and a non-transitory computer readable medium comprising instructions that, when executed by the at least one processor, cause the system to perform a method according to any one of claims 1-13.

15. A non-transitory computer-readable medium storing instructions that, when executed by at least one processor, cause a computing device to perform a method according to any one of claims 1-13.
